(19) Europäisches Patentamt
European Patent Office
Office européen des brevets

(11) EP 2 793 942 B1

(12) # FASCICULE DE BREVET EUROPEEN

(45) Date de publication et mention de la délivrance du brevet:
**15.04.2020 Bulletin 2020/16**

(21) Numéro de dépôt: **12819106.1**

(22) Date de dépôt: **21.12.2012**

(51) Int Cl.:
***A61K 39/395*** (2006.01) ***C07K 16/28*** (2006.01)
***A61K 31/282*** (2006.01) ***A61K 31/337*** (2006.01)
***A61P 35/00*** (2006.01) ***A61K 33/00*** (2006.01)

(86) Numéro de dépôt international:
**PCT/FR2012/053067**

(87) Numéro de publication internationale:
**WO 2013/093379 (27.06.2013 Gazette 2013/26)**

(54) **NOUVELLES COMPOSITIONS PHARMACEUTIQUES COMPRENANT UN ANTICORPS LIANT LE RECEPTEUR HUMAIN DE L'HORMONE ANTI-MULLERIENNE DE TYPE II**

NEUARTIGE PHARMAZEUTISCHE ZUSAMMENSETZUNGEN MIT EINEM DEN MENSCHLICHEN ANTI-MÜLLER-HORMONREZEPTOR TYP II BINDENDEN ANTIKÖRPER

NOVEL PHARMACEUTICAL COMPOSITIONS COMPRISING AN ANTIBODY WHICH BINDS THE HUMAN ANTI-MULLERIAN HORMONE RECEPTOR TYPE II

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2011 FR 1162424**

(43) Date de publication de la demande:
**29.10.2014 Bulletin 2014/44**

(73) Titulaires:
- **Laboratoire Français du Fractionnement et des Biotechnologies**
  **91958 Les Ulis (FR)**
- **Institut Régional du Cancer de Montpellier**
  **34090 Montpellier (FR)**
- **INSERM - Institut National de la Santé et de la Recherche Médicale**
  **75654 Paris Cedex 13 (FR)**
- **Université de Montpellier**
  **34090 Montpellier (FR)**

(72) Inventeurs:
- **GAUCHER, Christine**
  **F-59320 Equedin (FR)**
- **NAVARRO-TEULON, Isabelle**
  **F-34980 Saint Gely Du Fesc (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**EP-A1- 1 918 304 WO-A1-2011/141653**

- **GYNECOLOGIC ONCOLOGY GRP: "Intraperitoneal cisplatin and paclitaxel in ovarian cancer", NEW ENGLAND JOURNAL OF MEDICINE, vol. 354, no. 1, janvier 2006 (2006-01), pages 34-43, XP002676749, ISSN: 0028-4793**
- **PENSON R T ET AL: "Phase II study of carboplatin, paclitaxel, and bevacizumab with maintenance bevacizumab as first-line chemotherapy for advanced Mu"llerian tumors", JOURNAL OF CLINICAL ONCOLOGY 20100101 AMERICAN SOCIETY OF CLINICAL ONCOLOGY USA LNKD- DOI:10.1200/JCO.2009.22.7900, vol. 28, no. 1, 1 janvier 2010 (2010-01-01), pages 154-159, XP002676750, ISSN: 0732-183X**

EP 2 793 942 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **KERRI S BEVIS ET AL: "Anti-tumor activity of an anti-DR5 monoclonal antibody, TRA-8, in combination with taxane/platinum-based chemotherapy in an ovarian cancer model", GYNECOLOGIC ONCOLOGY, vol. 121, no. 1, 5 janvier 2011 (2011-01-05), pages 193-199, XP028164679, ISSN: 0090-8258, DOI: 10.1016/J.YGYNO.2010.11.046 [extrait le 2010-12-08]**


Remarques:

Le dossier contient des informations techniques présentées postérieurement au dépôt de la demande et ne figurant pas dans le présent fascicule.

## Description

La présente invention a pour objet de nouvelles compositions pharmaceutiques comprenant, à titre de substance active, un anticorps liant le récepteur humain de l'hormone anti-mullérienne de type II (AMHR-II), ainsi que les utilisations thérapeutiques de ces compositions.

L'hormone anti-mullérienne humaine est une glycoprotéine de 560 acides aminés, membre de la famille des TGF-$\beta$. C'est une hormone émise par les cellules de Sertoli du testicule fœtal, qui provoque la dégénérescence du canal de Müller. Elle est exprimée chez l'adulte dans les cellules de Sertoli et de Leydig (testicule) et les cellules de la granulosa (ovaire). Elle joue un rôle dans l'activité de l'ovaire adulte de régulation de la folliculogenèse.

Le récepteur de l'hormone anti-mullérienne de type II (AMHR-II) est un peptide de 573 acides aminés et possède une activité sérine-thréonine kinase. Il est impliqué dans la régression du canal de Müller associée au développement du système de reproduction de l'homme. Le canal de Müller s'atrophie chez l'homme où il ne forme que l'utricule prostatique et l'hydatide sessile, mais persiste chez la femme où il est à l'origine des trompes, de l'utérus et de la plus grande partie du vagin. Ce récepteur est fréquemment exprimé sur les cellules épithéliales tumorales d'ovaires humaines.

La demande internationale WO 2008/053330 décrit un anticorps monoclonal murin 12G4 dirigé contre AMHR-II pour le traitement des cancers ovariens.

La demande internationale WO 2011/141653 décrit des anticorps humanisés 12G4 mutés, ou des fragments de ceux-ci, possédant une affinité au moins égale à celle de l'anticorps chimérique correspondant non muté, une spécificité vis-à-vis du récepteur AMHR-II, et ne déclenchant pas de réaction immune.

La présente invention a pour but de proposer une alternative thérapeutique avantageuse aux patients atteints d'une pathologie liée au récepteur humain de l'hormone anti-mullérienne de type II (AMHR-II).

Une pathologie associée au récepteur de type II de l'hormone anti-mullérienne humaine (AMHR-II) peut notamment être : le cancer ovarien, en particulier le cancer de l'ovaire métastatique, le cancer séreux, l'hypernéphrome, l'endométrioïde, l'épithélium colloïde, elle peut aussi être : le cancer de la prostate, le cancer des cellules germinales, le cancer de l'endomètre, la tumeur maligne mullérienne mixte de l'utérus, le leiomyosarcome, et le sarcome stromal de l'endomètre.

Le cancer ovarien est la cause principale des cancers gynécologiques et est la cinquième cause de mortalité par cancer chez la femme. Ses trois origines histologiques sont les suivantes :

- l'épithélium de surface (tumeur épithéliale avec différents sous types) qui représente 85-90 % des cancers ovariens,
- les cordons sexuels / stroma (tumeur de la granulosa (3% des cancers ovariens totaux)), qui représentent environ 10 % des tumeurs ovariennes,
- les cellules germinales qui représentent 5 % des cancers ovariens.

Il est généralement asymptomatique pendant les premiers stades ce qui lui vaut le surnom de « silent killer » (La Marca A., Volpe A. The Anti-Mullerian hormone and ovarian cancer. Human Reproduction Update, Vol.13, No.3 pp. 265-273, 2007).

Il existe quatre stades et pronostic (classification FIGO : Fédération internationale de Gynécologie et d'Obstétrique) pour lesquels le taux de survie diminue fortement dès le stade 2:

Stade I: Tumeur limitée aux ovaires (taux de survie à 5ans: 90-70%),
Stade II: Tumeur dans un ou deux ovaires avec extension pelvienne (taux de survie à 5ans : 70-40%),
Stade III: Tumeur dans un ou deux ovaires avec extension extra pelvienne (taux de survie à 5ans : 20%),
Stade IV: Métastases à distance à l'exclusion des métastases péritonéales (taux de survie à 5ans : <10%),

(Fauci, Braunwald et al. Principles of internai medicine. Harrison's 17th edition / National Cancer Institute cancer.gov / CNGOF (collèges national des gynécologues et obstétriciens français).

En ce qui concerne le cancer ovarien, les principales stratégies utilisées pour le traitement sont la chirurgie et la chimiothérapie, en particulier en première ligne, tel qu'un mélange carboplatine et paclitaxel.

Des anticorps monoclonaux ont également été récemment développés tels que le cetuximab, qui est dirigé contre le récepteur du facteur de croissance épidermique (EGFR, Ozols R. F. et al., Focus on epithelial ovarian cancer, Cancer Cell. 2004, Jan ; 5(1):19-24). D'autres anticorps monoclonaux sont actuellement en phase III, tels que l'abagovomab dirigé contre le CA-125, l'avastin dirigé contre le facteur de croissance endothéliale vasculaire (VEGF-A), ou le farletuzumab, dirigé contre le récepteur alpha folique (FRA).

La présente invention a pour but de proposer une thérapie dirigée contre une cible différente des cibles des anticorps actuellement développés. L'invention présente l'avantage de proposer un traitement contre différentes maladies associées à AMHR-II. De plus, dans le cas du cancer de l'ovaire, l'invention offre la possibilité d'une thérapie plus efficace que la thérapie de référence pour réduire le volume tumoral, permettant ainsi une amélioration plus rapide de l'état du patient.

Ce but est réalisé grâce à une composition selon l'invention.

La présente invention a pour objet une composition pharmaceutique comprenant, à titre de substance active, en association avec un véhicule pharmaceutiquement acceptable,

- un agent anti-cancéreux, et

un anticorps liant le récepteur de type II de l'hormone anti-mullérienne humaine (AMHR-II).

Dans l'invention, le terme « anticorps » se réfère à une immunoglobuline, protéine multimérique constituée de 4 chaînes, soit 2 chaînes légères et 2 chaînes lourdes, comprenant chacune une région variable et une région constante. Plus précisément, chaque chaîne légère est constituée d'une région variable ($V_L$) et d'une région constante ($C_L$). Chaque chaîne lourde est constituée d'une région variable ($V_H$) et d'une région constante constituée de trois domaines constants $C_{H1}$, $C_{H2}$ et $C_{H3}$. Les domaines $C_{H2}$ et $C_{H3}$ composent le domaine Fc. La région variable de la chaîne légère est constituée de trois régions déterminant la reconnaissance de l'antigène (CDR) entourées de quatre domaines charpentes. Le repliement tridimensionnel de la région variable est tel que les 3 CDR sont exposés du même côté de la protéine et permettent la formation d'une structure spécifique reconnaissant un antigène déterminé.

Un « agent anti-cancéreux » est défini comme toute molécule pouvant soit interférer avec la biosynthèse de macromolécules (ADN, ARN, Protéines...) soit inhiber la prolifération cellulaire, soit entrainer la mort cellulaire par apoptose ou cytotoxicité par exemple. Parmi les agents anti-cancéreux, on peut citer les agents alkylants, les inhibiteurs de topoisomérase et agent intercalants, les anti-métabolites, les agents scindants, les agents interférants avec la tubuline, les anticorps monoclonaux.

Un « véhicule pharmaceutiquement acceptable » se réfère à un matériel non toxique qui est compatible avec un système biologique tel qu'une cellule, une culture cellulaire, un tissu ou un organisme.

Selon un aspect particulier, l'invention a pour objet une composition pharmaceutique comprenant, à titre de substance active, en association avec un véhicule pharmaceutiquement acceptable, un agent anti-cancéreux et un anticorps liant AMHR-II, dans laquelle ledit anticorps liant AMHR-II est un anticorps polyclonal.

Le terme « anticorps polyclonal » désigne un mélange d'anticorps, susceptibles de reconnaître des déterminants antigéniques différents d'une protéine cible.

Selon un autre aspect particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle ledit anticorps est un anticorps monoclonal, et de préférence un anticorps monoclonal 12G4 chimérique ou humanisé.

Un « anticorps monoclonal » est défini comme un anticorps ne reconnaissant qu'un seul déterminant antigénique de la protéine cible.

Par « anticorps monoclonal chimérique », on entend un anticorps dont les régions variables des chaînes légères et des chaînes lourdes appartiennent à une espèce différente de celle des régions constantes des chaînes légères et des chaînes lourdes. Par extension ou usage un anticorps chimérique fait référence à un anticorps avec des parties constantes d'origine humaine.

Les anticorps chimériques selon l'invention peuvent être préparés en utilisant les techniques de recombinaison génétique. Par exemple, un anticorps chimérique peut être réalisé en construisant un gène chimère comprenant une séquence nucléotidique d'ADN complémentaire (ADNc) ou génomique avec introns codant pour la région variable de la chaîne lourde d'un anticorps monoclonal murin, liée à une séquence nucléotidique codant pour la région constante de la chaîne lourde d'un anticorps humain, et en construisant un gène chimère comprenant une séquence nucléotidique codant pour la région variable de la chaîne légère d'un anticorps monoclonal murin, liée à une séquence nucléotidique codant pour la région constante de la chaîne légère d'un anticorps humain. En transfectant lesdits gènes chimères, par fusion de protoplastes ou toute autre technique, dans une lignée cellulaire, de myélome murin par exemple, on obtient la production d'anticorps chimériques souris-homme par les cellules transformées. C'est le document Morrison et al., Proc. Natl. Acad. Sci. U.S.A., 81, pp. 6851-55 (1984) qui a décrit pour la première fois la préparation de tels anticorps. Les documents Boulianne, G. L. et al., Nature, 312:643-646 (1984), Sun, L.K., et al., Proc. Natl. Acad. Sci. USA (1984), 214-218, US 4,816,567, US 6,331,415, US 6,808,901 et EP 125023 pourront également être utilisés comme référence par l'homme du métier, de même que Bobrzecka, K., et al, Immunology Letters 2, pp 151-155 qui décrit une procédure de fractionnement des ponts disulfures interchaînes des immunoglobulines suivie par un réarrangement ordonné de ces mêmes ponts disulfures afin d'obtenir des anticorps formés de fragments Fab de lapin et de fragments Fc humain.

Une autre approche de préparation d'anticorps chimériques, telle que décrite dans le document FR 2 641 468, peut être de greffer des fragments Fab' d'un anticorps monoclonal murin sur des immunoglobulines polyclonales humaine, notamment IgG, ou sur des fragments Fc, ceci à l'aide d'un agent de couplage, par exemple un diimide. Des anticorps chimériques de type Ig-Fab' (également noté Fab'-Ig), Fc-Fab' ou (Fab')2 peuvent ainsi être obtenus. De tels anticorps chimériques sont caractérisés par la greffe de l'ensemble du fragment Fab', et non seulement des parties variables.

Alternativement, d'autres auteurs ont décrit l'obtention d'anticorps chimériques monovalents par greffage de fragments Fab' d'anticorps polyclonaux sur des IgG ou sur des fragments Fc (G.T. Stevenson et al, Med. Oncol. & Tumor, 1985, Pharmacother, vol. 1, n°4, 275-278, 1984).

La recombinaison homologue *in vivo* entre les portions des gènes codant pour les régions constantes des chaînes légères et des chaînes lourdes d'une immunoglobuline murine par des portions des gènes codant pour les régions constantes des chaînes légères et des chaînes lourdes d'une immunoglobuline humaine est également un moyen qui peut être utilisé afin d'obtenir de tels anticorps (US 5,204,244 ou US 5,202,238). Cette liste n'est pas exhaustive.

Par « anticorps monoclonal humanisé », on entend un anticorps, dont tout ou partie des séquences des régions impliquées dans la reconnaissance de l'antigène (les régions hypervariables (CDR : Complementarity Determining Region), et parfois certains acides aminés des régions FR (régions Framework)), sont d'origine non-humaine (de préférence murine) tandis que les séquences des régions constantes et des régions variables non-impliquées dans la reconnaissance de l'antigène sont d'origine humaine.

Les anticorps humanisés selon l'invention peuvent être préparés par des techniques bien connues, telles que celle décrite pour la première fois dans le document Jones et al., Nature, 1986, 321-522-525. Il s'agissait du remplacement des régions hypervariables (CDRs) d'un anticorps humain par des régions hypervariables d'origine murine, à la fois au niveau des chaînes légères mais aussi des chaînes lourdes. Cette technique, aujourd'hui bien connue de l'homme de l'art sous le nom de «CDR grafting» a été décrite dans de nombreux documents tels que Singer et al., J. Immun. 150:2844-2857 (1993), Riechman et al., Nature, vol 332, 323:326 (1988), ou encore les brevets US 5,225,539 ; US 5,585,089 ; EP 0682040 qui pourront également être utilisés comme référence. La plupart des anticorps humanisés réalisés par greffage des régions CDRs présentent néanmoins une affinité réduite par rapport à un anticorps murin, ceci en raison du rôle majeur de certains acides aminés des régions charpentes dans le positionnement spatial des acides aminés non humains incluant les CDRs ainsi que dans la liaison à l'antigène. C'est pourquoi aujourd'hui l'homme du métier remplace dans l'Ig receveuse humaine, bien souvent, non seulement les CDRs, mais aussi les résidus des régions charpentes susceptibles de contribuer au site de liaison de l'antigène.

Une autre technique qui permet d'humaniser des anticorps est la technique de greffage des résidus déterminants spécifiques (Specificity Determining Région, SDR), qui consiste à greffer non plus l'ensemble des régions CDRs, mais uniquement les régions SDRs de l'anticorps non-humain dans les régions variables humaines (Tamura et al., J Immunol. 2000 ; 164 : 1432-41). Les régions SDRs sont définies comme les régions des CDRs en contact direct avec l'antigène (Padlan et al. (1995), FASEB J. 9: 133-139). Cette technique nécessite donc l'identification des SDRs. Cela peut se faire, par exemple, par une détermination de la structure 3D du complexe antigène-anticorps, en utilisant la base de données des SDRs déjà identifiés (http://paradox.harvard.edu/sdr), ou bien grâce à des comparaisons des séquences variables humaines à celles de l'espèce non-humaine, ceci à l'aide de logiciels informatiques tels que DomainGapAlign, CLUSTALW2, CLUSTALX, BLAST ou FASTA.

Une autre alternative pour obtenir des anticorps humanisés consiste à greffer des régions dites « CDRs abrégés » (Grafting of abbreviated CDRs). Il s'agit de la greffe des régions SDRs et de quelques résidus adjacents, en amont et en aval de la séquence. Les documents De Pascalis et al., The Journal of Immunology, 2002, 169: 3076-3084 ; Kashmiri Syed V.S et al., Methods, Volume 36, Issue , May 2005, Pages 25-34 pourront être utilisés comme référence.

La technologie d'humanisation composite développée par Antitope (WO 2006082406) est une technique de CDR grafting qui considère les régions charpente de façon indépendante et vise à choisir les équivalents humains séparément de tel sorte que la présentation des résidus en interaction à l'antigène soient mieux conservés dans leur orientation.

La technique dite de resurfaçage « variable domaine resurfacing », aussi nommée « veneering » telle que développée par ImmunoGen (US 5,639,641) ou Xoma (EP 0571613, US 5,766,886, US 5,770,196, US 5,821,123, US 5,869,619) peut également être utilisée. Cette technologie consiste à donner un « profil » humain à un domaine variable de souris en remplaçant les résidus exposés à la surface dans les régions charpentes des anticorps murins par les résidus habituellement trouvés à la surface des anticorps humains. Les documents Roguska et al., Proc Natl Acad Sci USA 1994 ; Mark G. E. et al (1994) in Handbook of Experimental Pharmacology vol. 113: The pharmacology of monoclonal Antibodies, Springer-Verlag, pp 105-134 pourront également servir de référence.

La plateforme Germliner™ développée par AvantGen peut également être utilisée (http://www.avantgen.com/AvantGensTechnologiesandServices.pdf). Cela permet d'obtenir des anticorps humanisés dans lesquels seuls des CDR3 sont d'origine non-humaine.

Cette liste n'est pas exhaustive. L'obtention desdits anticorps humanisés sera, de plus, préférentiellement couplée à un processus de maturation d'affinité.

L'obtention de l'anticorps monoclonal 12G4 humanisé est décrite en détail dans la demande internationale WO 2011/141653. Les anticorps décrits dans l'invention sont isolés et purifiés. Ces anticorps sont matures, c'est-à-dire qu'ils possèdent une structure tridimensionnelle ad hoc leur permettant de reconnaître l'antigène, et possèdent toutes les modifications post-traductionnelles essentielles à leur reconnaissance antigénique, notamment la glycosylation et la formation de ponts disulfures intra et inter moléculaires.

Selon un autre aspect, l'invention concerne un fragment d'un anticorps monoclonal 12G4 humanisé muté tel que défini ci-dessus, choisi parmi le groupe de fragments constitué de : Fc, Fab'-SH, Fd, Fv, Fab, F(ab')2, Fab', dsFv, scFv, sc(Fv)2, diabody, triabody ou tetrabody ou encore nanobody.

Selon un aspect encore plus particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'anticorps monoclonal 12G4 chimérique ou humanisé est muté, et comprend au moins une mutation dans la chaîne légère et/ou lourde.

Selon un aspect encore plus particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'anticorps monoclonal 12G4 chimérique ou humanisé est muté, comprend au moins une mutation dans la chaîne légère et/ou lourde, et possède une affinité pour AMHR-II caractérisée par un $K_D$ préférentiellement inférieur à $10^{-7}$ M, notamment inférieur à $10^{-8}$ M, en particulier compris de $10^{-9}$ M à $10^{-11}$ M.

Plus particulièrement, la présente invention a pour objet une composition pharmaceutique comprenant, à titre de substance active, en association avec un véhicule pharmaceutiquement acceptable, un agent anti-cancéreux, et un anticorps monoclonal liant AMHR-II, dans laquelle ledit anticorps monoclonal est un anticorps humanisé 12G4, ou un fragment d'anticorps monoclonal humanisé 12G4, ledit anticorps monoclonal humanisé 12G4 étant muté, et comprend au moins une mutation dans la chaîne légère et/ou lourde, ledit anticorps muté possédant une affinité pour le récepteur de type II de l'hormone anti-mullérienne humaine (AMHRII) caractérisée par un $K_D$ préférentiellement inférieur à $10^{-7}$ M, notamment inférieur à $10^{-8}$ M, en particulier compris de $10^{-9}$ M à $10^{-11}$ M.

Par « anticorps monoclonal humanisé 12G4 muté», on entend un anticorps monoclonal humanisé 12G4 dans lequel au moins une mutation a été effectuée dans la région variable de la chaîne légère et/ou la région constante de la chaîne légère et/ou la région variable de la chaîne lourde ou la région constante de la chaîne lourde. Un anticorps humanisé 12G4 muté, dans une composition selon l'invention, possède une affinité au moins égale à celle de l'anticorps chimérique correspondant non muté, une spécificité vis-à-vis de AMHR-II et ne déclenche pas de réaction immune.

De façon encore plus particulière, la présente invention a pour objet une composition pharmaceutique comprenant, à titre de substance active, en association avec un véhicule pharmaceutiquement acceptable, un agent anti-cancéreux, et un anticorps monoclonal liant AMHR-II, dans laquelle ledit anticorps monoclonal humanisé muté comprend ou est constitué :

a) d'une chaîne légère comprenant ou constituée :

- d'une région variable dont la séquence en acides aminés est représentée par SEQ ID NO:1 ou SEQ ID NO:2, et
- d'une région constante dont la séquence en acides aminés est représentée par SEQ ID NO:3 ou par une séquence présentant au moins 80% d'homologie avec la SEQ ID NO:3

b) d'une chaîne lourde comprenant ou constituée :

- d'une région variable dont la séquence en acides aminés est représentée par SEQ ID NO: 4, ou SEQ ID NO: 5, et
- d'une région constante dont la séquence en acides aminés est représentée par SEQ ID NO: 6 ou par une séquence présentant au moins 80% d'homologie avec la SEQ ID NO:6,

dans laquelle l'anticorps monoclonal humanisé 12G4 comprend au moins une mutation dans la chaîne légère et/ou lourde, et présente un $K_D$ pour le récepteur de type II de l'hormone anti-mullérienne humaine (AMH-II) préférentiellement inférieur à $10^{-7}$ M, notamment inférieur à $10^{-8}$ M, en particulier compris de $10^{-9}$ M à $10^{-11}$ M. Selon un aspect encore plus particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'anticorps monoclonal 12G4 chimérique ou humanisé *est* muté, et comprend au moins une mutation dans la chaîne légère et/ou lourde.

Les références des séquences en acides aminés des différentes parties des anticorps sont rassemblées dans le tableau suivant :

| | Références de la séquence dans la présente invention | Référence de la séquence dans la demande WO 2011/141653 |
|---|---|---|
| Anticorps 12G4 humanisé, chaîne légère, région variable, sans leader | *SEQ ID NO:1* | *SEQ ID NO:2* |

(suite)

| | Références de la séquence dans la présente invention | Référence de la séquence dans la demande WO 2011/141653 |
|---|---|---|
| Anticorps 12G4 humanisé, chaîne légère, région variable, avec leader | *SEQ ID NO:2* | *SEQ ID NO:4* |
| Anticorps 12G4 humanisé, chaîne légère, région constante, | *SEQ ID NO:3* | *SEQ ID NO:6* |
| Anticorps 12G4 humanisé, chaîne lourde, région variable, sans leader | *SEQ ID NO:4* | *SEQ ID NO: 8* |
| Anticorps 12G4 humanisé, chaîne lourde, région variable, avec leader | *SEQ ID NO:5* | *SEQ ID NO: 10* |
| Anticorps 12G4 humanisé, chaîne lourde, région constante | *SEQ ID NO:6* | *SEQ ID NO: 12* |
| Anticorps 3C23K, chaîne légère, région variable, sans leader | *SEQ ID NO:7* | *SEQ ID NO: 34* |
| Anticorps 3C23K, chaîne légère, région variable, avec leader | *SEQ ID NO:8* | *SEQ ID NO: 36* |
| Anticorps 3C23K, chaîne lourde, région variable, sans leader | *SEQ ID NO:9* | *SEQ ID NO: 38* |
| Anticorps 3C23K, chaîne lourde, région variable, avec leader | *SEQ ID NO:10* | *SEQ ID NO: 40* |
| Anticorps 3C23K, chaîne légère, sans leader | *SEQ ID NO:11* | SEQ ID NO:82 |
| Anticorps 3C23K, chaîne légère, avec leader | *SEQ ID NO:12* | SEQ ID NO:84 |
| Anticorps 3C23K, chaîne lourde, sans leader | *SEQ ID NO:13* | SEQ ID NO:86 |
| Anticorps 3C23K, chaîne lourde, avec leader | *SEQ ID NO:14* | SEQ ID NO:88 |

De façon encore plus particulière, la présente invention a pour objet une composition pharmaceutique comprenant, à titre de substance active, en association avec un véhicule pharmaceutiquement acceptable, un agent anticancéreux, et un anticorps monoclonal liant AMHR-II, dans laquelle ledit anticorps monoclonal humanisé muté comprend ou est constitué :

a) d'une chaîne légère comprenant ou constituée :

- d'une région variable dont la séquence en acides aminés est représentée par SEQ ID NO:7 ou SEQ ID NO:8,
- d'une région constante dont la séquence en acides aminés est représentée par SEQ ID NO:3

b) d'une chaîne lourde comprenant ou constituée :

- d'une région variable dont la séquence en acides aminés est représentée par SEQ ID NO:9 ou SEQ ID NO:10
- d'une région constante dont la séquence en acides aminés est représentée par SEQ ID NO:6

De façon encore plus particulière, la présente invention a pour objet une composition pharmaceutique comprenant, à titre de substance active, en association avec un véhicule pharmaceutiquement acceptable, un agent anticancéreux, et un anticorps monoclonal liant AMHR-II, dans laquelle ledit anticorps monoclonal humanisé muté comprend ou est constitué :

a) d'une chaîne légère constituée de la séquence en acides aminés représentée par SEQ ID NO: 11 (sans leader) ou SEQ ID NO: 12 (avec leader), et

b) d'une chaîne lourde comprenant ou constituée par la séquence en acides aminés représentée par SEQ ID NO: 13 (sans leader), ou SEQ ID NO: 14 (avec leader),

De façon encore plus particulière, la présente invention a pour objet une composition pharmaceutique comprenant, à titre de substance active, en association avec un véhicule pharmaceutiquement acceptable, un agent anticancéreux, et un anticorps monoclonal liant AMHR-II, dans laquelle ledit anticorps monoclonal humanisé muté est produit par le clone 3C23K.

L'anticorps monoclonal humanisé muté produit par le clone 3C23K est décrit en détail dans la demande internationale WO 2011/141653. Par référence à l'anticorps monoclonal humanisé 12G4, l'anticorps 3C23K possède les mutations de l'anticorps 3C_23, ainsi qu'une mutation supplémentaire, dans le CDR de la région variable de la chaîne légère (E184K) dans laquelle un acide glutamique est remplacé par une lysine, c'est-à-dire le remplacement d'un acide aminé acide par un acide aminé basique ayant par conséquent une charge totalement différente puisque de signe opposée, présente pourtant toujours une activité mais surtout une affinité sensiblement meilleure que celle de l'anticorps humanisé 12G4 non muté, et supérieure à celle de l'anticorps chimérique 12G4 non muté, et ne provoque pas de réaction immune.

L'anticorps monoclonal humanisé muté produit par le clone 3C23K présente une fucosylation d'une teneur d'environ 50%.

Selon un autre aspect, la présente invention a pour objet une composition pharmaceutique comprenant, à titre de substance active, en association avec un véhicule pharmaceutiquement acceptable, un agent anti-cancéreux, et un anticorps monoclonal liant AMHR-II, dans laquelle ledit anticorps monoclonal humanisé muté est produit par un clone décrit dans la demande WO 2011/141653 et choisi dans le groupe constitué par: 3C_23, 6B_78, 4C_35 et 5B_42.

Dans un mode de réalisation particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle ledit anticorps est un anticorps recombinant produit par transgénèse animale.

Cet anticorps recombinant peut ainsi être produit par toute technique connue de l'homme du métier, par exemple par recombinaison dans une cellule hôte, transformée avec un ou des vecteur(s) qui permettent l'expression et/ou la sécrétion des séquences nucléotidiques codant pour la chaîne lourde et/ou la chaîne légère de l'anticorps. Le vecteur comporte généralement un promoteur, des signaux d'initiation et de terminaison de la traduction, ainsi que des régions appropriées de régulation de la transcription. Il est maintenu de façon stable dans la cellule hôte et peut éventuellement posséder des signaux particuliers qui spécifient la sécrétion de la protéine traduite. Ces différents éléments sont choisis et optimisés par l'homme du métier en fonction de l'hôte cellulaire utilisé. De tels vecteurs sont préparés par des méthodes couramment utilisées par l'homme du métier, et les clones résultant peuvent être introduits dans un hôte approprié par des méthodes standard. L'hôte cellulaire peut être choisi parmi des systèmes procaryotes ou eucaryotes, par exemple les cellules bactériennes mais également les cellules de levure ou les cellules animales, en particulier les cellules de mammifères. Les cellules de mammifère préférées pour la production de l'anticorps monoclonal sont la lignée de rat YB2/0, la lignée de hamster CHO, PER.C6TM (Crucell), 293, K562, NS0, SP2/0, BHK ou COS. On peut également utiliser des cellules d'insectes. Un autre mode de production est l'expression de l'anticorps recombinant dans des organismes transgéniques, par exemple dans les plantes ou surtout dans le lait d'animaux transgéniques tels que la lapine, la chèvre ou le porc. Selon un mode de réalisation préféré, l'anticorps est produit dans le lait de mammifères transgéniques non humains, modifiés génétiquement pour produire cette glycoprotéine. De préférence, il s'agit du lait d'une lapine ou d'une chèvre transgénique, de préférence dans le lait d'une chèvre transgénique. De manière avantageuse, l'anticorps produit par transgénèse animale, en particulier dans les glandes mammaires d'une chèvre transgénique, présente une glycosylation avec un taux de galactosylation élevé, par exemple supérieur 70%.

De façon particulière, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'agent anti-cancéreux est du paclitaxel ou un sel de platine choisi parmi le groupe constitué par : oxaloplatine, cisplatine et carboplatine.

L'agent anti-cancéreux peut aussi être choisi parmi des agents chimiothérapeutiques autres que les sels de platine, des petites molécules, des anticorps monoclonaux ou encore des peptibody anti-angiogénèse.

Parmi les agents chimiothérapeutiques autres que les sels de platine, on trouve les agents intercalant (blocage replication ADN et transcription), tels que les anthracyclines (doxorubicin, pegylated liposomal doxorubcin) les inhibiteurs de la topoisomérase (camptothecine et dérivés : Karenitecin, Topotecan, irinotecan),ou encore le SJG-136, les inhibiteurs de d'histone deacetylase (vorinostat, belinostat, Valproic acid), les agents alkylants (bendamustine, glufosfamide, Temozolomide), les alcaloïdes végétaux anti-mitotiques, tels que les taxanes (Docetaxel, paclitaxel), les vinca-alcaloïdes (vinorelbine), les epothilones (ZK-Epothilone, Ixabepilone), les anti-métabolites (Gemcitabine, Elacytarabine, Capécitabine), les inhibiteurs de kinesin spindle protein (KSP) (Ispinesib), la trabectedine ou encore l'ombrabuline (combretastatin A-4 derivative).

Parmi les petites molécules, on trouve les poly(ADP-ribose)polymerases (PARP) inhibiteurs : olaparib, iniparib, veliparib, rucaparib, CEP-9722, MK-4827, BMN-673, les inhibiteurs de kinases, tels que les inhibiteurs de Tyrosine kinase (TKI) parmi lesquelles on peut citer, les molécules anti-VEGFR (sorafenib, sunitinib, cediranib, vandetanib, pazopanib, BIBF 1120, semaxanib, Cabozantinib, motesanib), les molécules anti-HER2/EGFR (erlotinib, gefitinib, lapatinib), les molécules anti-PDGFR (imatinib, BIBF 1120), les molécules anti-FGFR (BIBF 1120), les inhibiteurs de aurora kinase/tyrosine kinase (ENMD-2076), les Src/Abl kinase inhibiteur (Saracatinib), ou encore la Perifosine, le Temsirolimus (mTOR inhibiteur), alvocidib (cyclin-dependent kinase inhibiteur), le Volasertib (inhibiteur de PLK1 (polo-like kinase 1) proteine, LY2606368 (inhibiteur de checkpoint kinase 1(chk 1), GDC-0449 (Hedgehog Pathway Inhibiteur), Zibotentan (antagoniste du ETA-récepteur), Bortezomib, Carfilzomib (proteasome inhibiteur), des cytokines telles que IL-12, IL-18, IL-21, INF-alpha, INF-gamma.

Parmi les anticorps, on peut citer, l'anti-VEGF : bevacizumab, l'anti-VEGFR : ramucirumab, les anti-HER2/EGFR: trastuzumab, pertuzumab, cetuximab, panitumumab, MGAH22, matuzumab, l'anti-PDGFR alpha: IMC-3G3, l'anti-Folate Récepteur : farletuzumab, l'anti-CD27: CDX-1127, l'anti-CD56: BB-10901, l'anti-CD105 : TRC105, l'anti-CD276 : MGA271, l'anti-AGS-8: AGS-8M4, l'anti-DR5 : TRA-8, l'anti-HB-EGF : KHK2866, les anti-mesothéline : amatuximab, BAY 94-9343 (immunotoxine), le catumaxomab (anticorps bispécifique EpCAM/CD3), l'anti-IL2R : daclizumab, l'anti-IGF-1R: ganitumab, l'anti-CTLA-4 : ipilimumab, l'anti-Lewis Y : Hu3S193, SGN-15 (immunotoxine), l'anti-CA125: oregovomab, l'anti-HGF : rilotumumab, l'anti-IL6 : siltuximab, l'anti-TR2 : tigatuzumab, l'anti-alpha5 beta1 intégrine : volociximab, l'anti-HB-EGF : KHK2866.

Les peptibody anti-angiogénèse sont choisis parmi l'AMG 386 et le CVX-241.

De façon plus particulière, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'agent anti-cancéreux est du carboplatine.

De façon encore plus particulière, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'anticorps monoclonal humanisé muté est produit par le clone 3C23K et l'agent anti-cancéreux est du carboplatine.

Dans un aspect particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'agent anti-cancéreux est du paclitaxel.

Dans un aspect plus particulier, l'invention a pour objet une composition pharmaceutique comprenant, à titre de substance active, en association avec un véhicule pharmaceutiquement acceptable, un agent anti-cancéreux, et un anticorps liant AMHR-II, dans laquelle l'anticorps monoclonal humanisé muté est produit par le clone 3C23K et l'agent anti-cancéreux est du paclitaxel.

Dans un aspect particulier, la présente invention a pour objet une composition pharmaceutique comprenant, à titre de substance active, en association avec un véhicule pharmaceutiquement acceptable, un agent anti-cancéreux, et un anticorps liant AMHR-II, dans une formulation destinée à une administration par voie intra-veineuse ou intra-péritonéale.

La composition pharmaceutique de l'invention peut être administrée par toute voie d'administration appropriée, par exemple par voie parentérale, orale, sublinguale, vaginale, rectale, transdermale, de préférence par injection intraveineuse, sous-cutanée ou intradermique. Une injection intramusculaire, intrapéritonéale, intrasynoviale, intrathécale ou intratumorale est aussi possible. Les injections peuvent être réalisées sous forme de bolus, ou par perfusion continue. Lorsque la composition d'anticorps et la composition d'agent anti-cancéreux sont administrées séparément, ces compositions peuvent être sous une forme d'administration identique ou différente.

Les préparations pour une administration parentérale peuvent inclure des solutions aqueuses ou non-aqueuses stériles, des suspensions ou des émulsions. Des exemples de solvants non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées sous forme de suspensions injectables, gels, huiles, comprimés, suppositoires, poudres, gélules, capsules, etc.

Selon un aspect particulier, l'invention a pour objet une composition pharmaceutique comprenant, à titre de substance active, en association avec un véhicule pharmaceutiquement acceptable, un agent anti-cancéreux et un anticorps liant AMHR-II, dans laquelle la quantité thérapeutiquement efficace d'anticorps administrée à un patient est comprise dans une gamme d'environ 0,07 mg à environ 35000 mg, de préférence d'environ 0,7 mg à environ 7000 mg, de préférence d'environ 0,7 mg à environ 1400 mg, de préférence d'environ 0,7 mg à environ 700 mg, et plus préférentiellement d'environ 0,7 mg à environ 70 mg.

Le dosage de la substance active dépend particulièrement du mode d'administration, et est aisément déterminé par l'homme de métier. Une quantité thérapeutiquement (dose unitaire) efficace d'anticorps peut varier de 0,01 mg/kg à 500 mg/kg, préférablement de 0,1 mg/kg à 500 mg/kg, préférablement de 0,1 mg/kg à 100 mg/kg, préférablement de 0,1 mg/kg à 20 mg/kg, préférablement de 0,1 mg/kg à 10 mg/kg, et plus préférablement de 1 mg/kg à 10 mg/kg, en une ou plusieurs administrations hebdomadaire, pendant plusieurs semaines ou mois. La dose unitaire efficace peut donc aisément être déduite d'une dose calculée pour un patient « moyen » sont le poids est de 70 kg.

Selon un autre aspect particulier, l'invention a pour objet une composition pharmaceutique comprenant, à titre de substance active, en association avec un véhicule pharmaceutiquement acceptable, un agent anti-cancéreux et un anticorps liant AMHR-II, dans laquelle la quantité thérapeutiquement efficace d'agent anti-cancéreux administrée à un patient est comprise dans une gamme d'environ 10 mg à environ 700 mg, de préférence dans une gamme d'environ 20 mg à environ 350 mg, et de préférence d'environ 110 mg.

Le dosage de l'agent anti-cancéreux dépend particulièrement du mode d'administration, et est aisément déterminé par l'homme de métier. Une quantité thérapeutiquement (dose unitaire) efficace peut varier de 0,2 $mg/m^2$ à 10 $g/m^2$, préférablement de 0,2 $mg/m^2$ à 1 $g/m^2$, préférablement de 2 $mg/m^2$ à 1 $g/m^2$, préférablement de 20 $mg/m^2$ à 1 $g/m^2$, et plus préférablement de 20 $mg/m^2$ à 0,5 $g/m^2$, en une ou plusieurs administrations hebdomadaire, pendant plusieurs semaines ou mois. La dose unitaire efficace peut donc être déduite d'une dose calculée pour un patient « moyen » sont la surface corporelle est d'environ 1,8 m2.

Selon un aspect encore plus particulier, l'invention a pour objet une composition pharmaceutique comprenant, à titre de substance active, en association avec un véhicule pharmaceutiquement acceptable, un agent anti-cancéreux et un anticorps liant AMHR-II, dans laquelle la quantité thérapeutiquement efficace d'agent anti-cancéreux administrée à un patient est d'environ 110 mg, et la quantité thérapeutiquement efficace d'anticorps administrée au patient est d'environ 70 mg.

L'invention a également pour objet une composition comprenant un agent anti-cancéreux et un anticorps liant le récepteur de type II de l'hormone anti-mullérienne humaine (AMHR-II), pour son utilisation comme médicament dans la prévention ou le traitement d'une pathologie associée au récepteur de type II de l'hormone anti-mullérienne humaine (AMHR-II).

Par traitement, on entend le moyen de soigner une pathologie déclarée, dont les symptômes sont visibles. Par prévention, on entend le moyen d'empêcher ladite pathologie de se déclarer.

Une pathologie associée au récepteur de type II de l'hormone anti-mullérienne humaine (AMHR-II) peut notamment être :

- le cancer ovarien, en particulier le cancer de l'ovaire métastatique, et ses différents sous-types, notamment : séreux, à cellules claires, endométrioïde, mucineux,
- le cancer des cellules germinales,
- le cancer de l'endomètre,
- la tumeur maligne mullérienne mixte de l'utérus,
- le leiomyosarcome,
- le sarcome stromal de l'endomètre
- le cancer de la prostate,
- le cancer du testicule.

Les tumeurs exprimant l'antigène AMHR-II sont préférentiellement ciblées, c'est-à-dire les tumeurs dans lesquelles on observe un niveau significatif de l'expression de l'antigène AMHR-II dans une cellule, de préférence sur la surface des cellules.

Conformément à l'invention, les deux agents thérapeutiques sont utilisés en combinaison afin de potentialiser les effets antiprolifératifs de l'un et de l'autre.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle ledit anticorps est un anticorps polyclonal.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle ledit anticorps est un anticorps monoclonal, et de préférence un anticorps monoclonal 12G4 chimérique ou humanisé.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle ledit anticorps est un anticorps humanisé 12G4 muté, ou un fragment d'anticorps monoclonal humanisé 12G4 muté, dans laquelle ledit anticorps monoclonal comprend au moins une mutation dans la chaîne légère et/ou lourde.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle ledit anticorps est un anticorps humanisé 12G4 muté, ou un fragment d'anticorps monoclonal humanisé 12G4 muté, dans laquelle ledit anticorps monoclonal comprend au moins une mutation dans la chaîne légère et/ou lourde et possède une affinité AMHR-II caractérisée par un $K_D$ préférentiellement inférieur à $10^{-7}$ M, notamment inférieur à $10^{-8}$ M, en particulier compris de $10^{-9}$ M à $10^{-11}$ M. Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle ledit anticorps est un anticorps humanisé 12G4 muté, ou un fragment d'anticorps monoclonal humanisé 12G4 muté, dans laquelle ledit anticorps monoclonal comprend au moins une mutation dans la chaîne légère et/ou lourde.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle ledit anticorps 12G4 comprend ou est constitué :

a) d'une chaîne légère comprenant ou constituée :

- d'une région variable dont la séquence en acides aminés est représentée par SEQ ID NO:1 ou SEQ ID NO:2, et
- d'une région constante dont la séquence en acides aminés est représentée par SEQ ID NO:3 ou présentant au moins 80% d'homologie avec la SEQ ID NO:3,

b) d'une chaîne lourde comprenant ou constituée :

- d'une région variable dont la séquence en acides aminés est représentée par SEQ ID NO: 4, ou SEQ ID NO: 5, et
- d'une région constante dont la séquence en acides aminés est représentée par SEQ ID NO: 6 ou par une séquence présentant au moins 80% d'homologie avec la SEQ ID NO:6,

dans laquelle l'anticorps monoclonal humanisé 12G4 est muté, comprend au moins une mutation dans la chaîne légère et/ou lourde, et présente un $K_D$ pour le récepteur de type II de l'hormone anti-mullérienne humaine (AMHR-II) préférentiellement inférieur à $10^{-7}$ M, notamment inférieur à $10^{-8}$ M, en particulier compris de $10^{-9}$ M à $10^{-11}$ M.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'anticorps monoclonal humanisé comprend ou est constitué

a) d'une chaîne légère comprenant ou constituée :

- d'une région variable dont la séquence en acides aminés est représentée par SEQ ID NO:7 ou SEQ ID NO:8 ,
- d'une région constante dont la séquence en acides aminés est représentée par SEQ ID NO: 3

b) d'une chaîne lourde comprenant ou constituée :

- d'une région variable dont la séquence en acides aminés est représentée par SEQ ID NO: 9 ou SEQ ID NO: 10, et
- d'une région constante dont la séquence en acides aminés est représentée par SEQ ID NO: 6.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'anticorps monoclonal humanisé 12G4 est produit par le clone 3C-23K.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'anticorps monoclonal humanisé 12G4 est un fragment de l'anticorps monoclonal humanisé 12G4 produit par le clone 3C-23K.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'anticorps est un anticorps recombinant produit par transgénèse animale.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle la pathologie associée au récepteur de type II de l'hormone anti-mullérienne humaine (AMHR-II) est le cancer, et particulièrement le cancer ovarien.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'agent anti-cancéreux est du paclitaxel ou un sel de platine choisi parmi le groupe constitué par : oxaloplatine, cisplatine, carboplatine.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'agent anti-cancéreux est du carboplatine.

Dans un aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'agent anti-cancéreux est du paclitaxel.

Dans un aspect particulier, l'invention a pour objet une composition pour son utilisation comme médicament dans la prévention ou le traitement d'une pathologie associée à AMHR-II, comprenant l'anticorps monoclonal 12G4 produit par le clone 3C23K et du carboplatine.

Dans un autre aspect particulier, l'invention a pour objet une composition pour son utilisation comme médicament dans la prévention ou le traitement d'une pathologie associée à AMHR-II, comprenant l'anticorps monoclonal 12G4 produit par le clone 3C23K et du paclitaxel.

Dans un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans une formulation destinée à une administration par voie intra-veineuse ou intra-péritonéale

Dans un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, l'anticorps monoclonal et l'agent anti-cancéreux étant destinés à une administration séparée, simultanée ou séquentielle.

L'anticorps et l'agent anti-cancéreux peuvent être combinés au sein d'une même composition pharmaceutique, ou être utilisés sous forme de compositions pharmaceutiques séparées, administrables de manière simultanée ou séquentielle. En particulier, les produits peuvent être administrés séparément, à savoir soit concomitamment, soit indépendamment, par exemple, avec un décalage dans le temps.

Plus particulièrement, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle l'anticorps et l'agent anticancéreux sont combinés au sein de la même composition pharmaceutique.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle la quantité thérapeutiquement efficace d'anticorps administrée à un patient est comprise dans une gamme d'environ 0,07 mg à environ 35.000 mg, de préférence d'environ 0,7 mg à environ 7000 mg, de préférence d'environ 0,7 mg à environ 1400 mg, de préférence d'environ 0,7 mg à environ 700 mg, et plus préférentiellement d'environ 0,7 mg à environ 70 mg.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle la quantité thérapeutiquement efficace d'agent anticancéreux administrée à un patient est comprise dans une gamme d'environ 10 mg à environ 700 mg, de préférence dans une gamme d'environ 20 mg à environ 350 mg, et de préférence d'environ 110 mg.

Selon un autre aspect particulier, l'invention a pour objet une composition pour son utilisation comme médicament dans la prévention ou le traitement d'une pathologie associée à AMHR-II, comprenant un agent anti-cancéreux et un anticorps liant AMHR-II, dans laquelle la quantité thérapeutiquement efficace d'anticorps administrée à un patient est d'environ 70 mg et la dose d'agent anticancéreux administrée au patient est d'environ 110 mg.

Dans un mode de réalisation préféré, le dosage d'agent anti-cancéreux, notamment le carboplatine ou le paclitaxel, est compris dans une gamme allant d'environ 0,01 mg / kg à environ 500 mg / kg, par exemple 0,1 mg / kg à 300 mg/ kg, soit d'environ 0.1 mg à 20 g par jour.

En variante, une dose de charge initiale plus élevée, suivie par une ou plusieurs doses inférieures peuvent aussi être administrées. En autre variante, une dose de charge initiale moins élevée, suivie par une ou plusieurs doses supérieures peuvent aussi être administrées.

Dans un mode de réalisation particulier, on peut utiliser l'anticorps anti-AMHR-II et le carboplatine dans un rapport anticorps/carboplatine compris dans une gamme entre environ 10/1 à environ 0,1/1, notamment entre environ 10/1 et environ 1/1, ou entre environ 1/1 à environ 0,1/1.

Dans un autre mode de réalisation particulier, on peut utiliser l'anticorps anti-AMHR-II et le paclitaxel dans un rapport anticorps/paclitaxel compris dans une gamme entre environ 10/1 à environ 0,1/1, notamment entre environ 10/1 et environ 1/1, ou entre environ 1/1 à environ 0,1/1

L'invention a par ailleurs pour objet un produit comprenant un anticorps liant le récepteur de type II de l'hormone anti-mullérienne humaine (AMHR-II) et un agent anti-cancéreux, sous la forme d'une préparation combinée, pour une utilisation simultanée, séquentielle ou séparée comme médicament destiné à prévenir ou traiter une pathologie associée au récepteur de type II de l'hormone anti-mullérienne humaine (AMHR-II), en particulier le cancer, et plus particulièrement le cancer ovarien.

Selon un aspect particulier, l'invention a pour objet un produit comprenant un anticorps liant le récepteur de type II de l'hormone anti-mullérienne humaine (AMHR-II) et un agent anti-cancéreux, sous la forme d'une préparation combinée, pour une utilisation simultanée, séquentielle ou séparée comme médicament destiné à prévenir ou traiter une pathologie associée au récepteur de type II de l'hormone anti-mullérienne humaine (AMHR-II), en particulier le cancer, et plus particulièrement le cancer ovarien, pour une utilisation simultanée de l'anticorps et de l'agent anti-cancéreux.

Selon un autre aspect particulier, l'invention a pour objet un produit comprenant un anticorps liant le récepteur de type II de l'hormone anti-mullérienne humaine (AMHR-II) et un agent anti-cancéreux, sous la forme d'une préparation combinée, pour une utilisation simultanée, séquentielle ou séparée comme médicament destiné à prévenir ou traiter une pathologie associée au récepteur de type II de l'hormone anti-mullérienne humaine (AMHR-II), en particulier le cancer, et plus particulièrement le cancer ovarien, pour une utilisation séquentielle de l'anticorps et de l'agent anti-cancéreux, dans laquelle l'anticorps est administré préalablement à l'agent anti-cancéreux.

Selon un autre aspect particulier, l'invention a pour objet un produit comprenant un anticorps liant le récepteur de type II de l'hormone anti-mullérienne humaine (AMHR-II) et un agent anti-cancéreux, sous la forme d'une préparation combinée, pour une utilisation simultanée, séquentielle ou séparée comme médicament destiné à prévenir ou traiter une pathologie associée au récepteur de type II de l'hormone anti-mullérienne humaine (AMHR-II), en particulier le cancer, et plus particulièrement le cancer ovarien, pour une utilisation séquentielle de l'anticorps et de l'agent anti-cancéreux, dans laquelle l'agent anti-cancéreux est administré préalablement à l'anticorps.

Les figures, tableaux et exemples suivants illustrent l'invention, sans en limiter la portée.

La figure 1A représente l'évolution des moyennes des volumes tumoraux, exprimés en mm3, selon l'axe des ordonnées, en fonction du nombre de jours comptés à partir du jour de l'injection des cellules tumorales. La figure 1B représente la courbe des médianes des volumes tumoraux en mm3, selon l'axe des ordonnées, en fonction du nombre de jours comptés à partir du jour de l'injection des cellules tumorales. Dans chacun des graphes, la courbe reliant les carrés pleins représente la moyenne des souris du groupe témoin, la courbe continue reliant les losanges représente le groupe des souris traitées par l'anticorps irrelevant LFB-R565, la courbe reliant les triangles pleins représente le groupe des souris traitées par l'anticorps 3C23K, la courbe continue reliant les ronds pleins représente le groupe des souris traitées par le carboplatine, la courbe en pointillés reliant les ronds pleins représente le groupe des souris traitées par le carboplatine et par l'anticorps irrelevant LFB-R565, la courbe en pointillés reliant les triangles pleins représente le groupe des souris traitées par le carboplatine et par l'anticorps 3C23K.

Les figures 2A à 2F représentent les courbes des volumes tumoraux individuels, par groupes. Pour chacun des graphes 2A à 2F, l'axe des abscisses représente le nombre de jours compté à partir du jour de l'injection des cellules tumorales, et l'axe des ordonnées représente le volume tumoral, chacune des courbes représente l'évolution du volume tumoral pour un animal. La figure 2A représente le groupe des souris témoin, la figure 2B représente le groupe des souris traitées par l'anticorps irrelevant LFB-R565, la figure 2C représente le groupe des souris traitées par l'anticorps 3C23K, la figure 2D représente le groupe des souris traitées par le carboplatine, la figure 2E représente le groupe des souris traitées par le carboplatine et par l'anticorps irrelevant, la figure 2F représente le groupe des souris traitées par le carboplatine et par l'anticorps 3C23K.

Les figures 3A, 3B et 3C représentent l'évolution des moyennes des volumes tumoraux (3A) ou des médianes des volumes tumoraux (3B) et le pourcentage de survie, dans 4 groupes de souris traités respectivement par l'anticorps 3C23K en monothérapie, du paclitaxel en monothérapie ou la combinaison de l'anticorps 3C23K et du paclitaxel. Une solution de NaCl était utilisée en tant que témoin. La figure 3A représente l'évolution des moyennes des volumes tumoraux, exprimés en mm3, selon l'axe des ordonnées, en fonction du nombre de jours comptés à partir du jour de l'injection des cellules tumorales. La figure 3B représente la courbe des médianes des volumes tumoraux en mm3, selon l'axe des ordonnées, en fonction du nombre de jours comptés à partir du jour de l'injection des cellules tumorales. La figure 3C représente le pourcentage respectif de survie pour les quatre groupes, selon l'axe des ordonnées, en fonction du nombre de jours comptés à partir du jour de l'injection des cellules tumorales. Dans les figures 3A à 3C : les courbes reliant les losanges correspondent au groupe traité par la solution témoin ; les courbes reliant les carrés correspondent au groupe traité par l'anticorps 3C23K en monothérapie ; les courbes reliant les points ronds correspondent au groupe traité par du paclitaxel ; les courbes reliant les triangles correspondent au groupe traité par la combinaison de l'anticorps 3C23K et du paclitaxel.

Les figures 4A, 4B, 4C et 4D représentent respectivement la courbe des volumes tumoraux individuels dans 4 groupes de souris traités respectivement par la solution témoin (4A), l'anticorps 3C23K en monothérapie (4B), du paclitaxel en monothérapie (4C), ou la combinaison de l'anticorps 3C23K et du paclitaxel (4D).

Le Tableau 1 présente un résumé du schéma de traitement.
Le Tableau 2 présente un résumé des données brutes de volume tumoral individuel.
Le Tableau 3 présente un résumé des données brutes de volume tumoral moyen et de la déviation standard (SD).
Le Tableau 4 présente les données brutes des volumes tumoraux médians et des rapports T/C.
Le Tableau 5 présente une analyse statistique des volumes tumoraux.
Le Tableau 6 présente un résumé des données brutes de poids corporel individuel.
Le Tableau 7 représente un résumé des données brutes de moyennes de poids corporel et de la déviation standard (SD).
Le tableau 8 représente un résumé des données brutes de poids corporel individuel.
Le tableau 9 représente un résumé des données brutes de volume tumoral individuel.
Le tableau 10 représente un résumé des données brutes de changement en moyenne de poids corporel.
Le tableau 11 représente un résumé des données brutes de moyennes des volumes tumoraux.
Le tableau 12 représente un résumé des données brutes des volumes tumoraux médians et des rapports T/C.
Le tableau 13 représente les résultats d'analyse statistique.
Le tableau 14 représente un résumé des données brutes de paramètre de survie
Le tableau 15 représente les paramètres de survie

## EXEMPLE

### Evaluation de l'activité de l'anticorps produit par le clone 3C23K (anticorps 3C23K), en monothérapie ou en association avec le carboplatine, dans un modèle de cancer ovarien humain Cov434-AMHRII Asc1a5 chez la souris femelle Swiss nude.

### 1. Protocole

Des souris femelle swiss nude (Harlan Laboratories) ont été injectées par voie sous-cutanée (s.c.) avec $7.10^6$ cellules de Cov434-AMHRII Asc1a5 (lignée cellulaire de cancer ovarien humain transfectée avec l'ADNc AMHRII)dans du matrigel (ratio 1:1) sous un volume de 150 µL au jour 0 (J0).

L'anticorps 3C23K a été évalué selon le schéma suivant : 2 fois par semaine pendant 6 semaines, pour un total de 12 injections a environ 10mg/kg/injection, ledit régime d'administration étant désigné ci-après par « Q3-4D12 ». Un autre groupe de souris a été traité par un anticorps irrelevant LFB-R565, administrés à environ 10 mg/kg/injection, selon le régime Q3-4D12.

Le carboplatine a été évalué, à une dose sous-optimale, soit environ 60 mg/kg/injection, selon le schéma suivant : une fois par semaine, durant 4 semaines, ledit régime d'administration étant désigné ci-après par « Q7D4 ».

Le carboplatine a également été évalué en combinaison avec l'anticorps 3C23K ou avec l'anticorps irrelevant LFB-R565. Le carboplatine a été administré à environ 60 mg/kg/injection selon le régime Q7D4 et l'anticorps 3C23K ou l'anticorps irrelevant LFB-R565 à environ 10 mg/kg/injection selon le régime Q3-4D12.

Les souris ont été randomisées à J11, quand le volume des tumeurs était compris entre 50 et 158 mm3, et les traitements ont commencé à J13 (9 souris par groupe). La description des dates de traitement est présentée dans le tableau 1.

Dans le cadre de l'évaluation de l'activité de la combinaison de l'anticorps 3C23K et du paclitaxel par rapport à celle de l'anticorps 3C23K en monothérapie ou du paclitaxel en monothérapie, l'anticorps 3C23K a été injecté selon le régime suivant : une fois par semaine pendant 4 semaines, pour un total de 4 injections, à une dose environ 10mg/kg/injection selon le régime Q7D4 ; le paclitaxel a été injecté selon le régime suivant : une fois par semaine pendant 4 semaine, pour un total de 4 injections, à une dose environ 15mg/kg/injection (le régime Q7D4).

Les souris ont été randomisées à J13, quand le volume des tumeurs était compris entre 58 et 150 mm3, et les traitements ont commencé à J14 (8 souris par groupe).

### a. Suivi des expériences in vivo

La mesure des tumeurs a été réalisée habituellement deux fois par semaine. Le volume tumoral (TV) a été calculé en utilisant la formule suivante, dans laquelle la longueur correspond au plus grand des diamètres tumoraux, la largeur correspond au plus petit des diamètres tumoraux et la hauteur tumorale: TV (mm3) = (longueur x largeur x hauteur) / 2

Les courbes de volume individuel des tumeurs ont été tracées.

D'autre part, pour chaque groupe, des courbes de croissance tumorale ont été tracées en utilisant la moyenne calculée des volumes tumoraux ou la médiane des volumes tumoraux.

Les animaux ont été sacrifiés lorsque les volumes de tumeurs atteignaient 2000 mm3 ou pour des raisons éthiques. Les courbes de moyennes et médianes ainsi que les analyses statistiques ont été arrêtés lorsque 20% des souris du groupe étaient mortes.

Dans une expérience avec 9 souris par groupe, les courbes et analyses ont donc été stoppées quand moins de 8 valeurs par groupe ont été obtenues (8 souris vivantes).

Dans une expérience avec 8 souris par groupe, les courbes et analyses sont arrêtées quand moins de 7 valeurs par groupe ont été obtenues (7 souris vivantes).

**b. Evaluation de l'efficacité du traitement**

L'inhibition de la croissance tumorale, définie comme le ratio entre les volumes tumoraux médians des souris traitées vis-à-vis des groupes contrôle traités (T/C) a été calculée ainsi :

$$T/C = (\text{moyenne TV du groupe traité} / \text{moyenne TV du groupe contrôle}) \times 100$$

Le National Cancer Institute utilisait les critères suivants pour évaluer l'activité antitumorale d'un produit (Bissery and al, 1991):

- T/C supérieur à 42%, le produit est considéré comme inefficace
- T/C entre 42% et 10%, le produit présente un effet antitumoral
- T/C inférieur à 10%, le produit est réellement efficace.

De plus, pour identifier si le traitement a un effet toxique, le poids des souris a été suivi individuellement une fois par semaine. La moyenne du poids corporel des souris a été calculée pour chaque groupe, jusqu'à ce que 20% des souris du groupe soient mortes.

**c. Analyses statistiques**

Les différences statistiques entre les différents groupes ont été analysées par comparaison ANOVA en utilisant le logiciel Statgraphics centurion XV.

La table ANOVA décompose la variance en deux composants : un composant inter-groupe et un composant intra-groupe. Le ratio F est le ratio entre l'estimation inter-groupe et l'estimation intra-groupe. Quand la valeur P du test F est supérieure ou égale à 0,05, il n'y a pas de différence statistiquement significative entre les moyennes des deux groupes, avec un niveau de confiance de 95%. Des valeurs P inférieures à 0,05 indiquent une différence significative entre les moyennes des deux groupes avec un niveau de confiance de 95%.

Les données brutes des analyses statistiques lors des expériences avec le ratio-F et la valeur P sont présentées en appendice pour toutes les expérimentations.

Un test de Kruskal-Wallis a également été réalisé. Quand la valeur P est supérieure ou égale à 0,05, il n'y a pas de différence statistiquement significative entre la médiane des deux groupes, avec un niveau de confiance de 95%. Des valeurs P inférieures à 0,05 indiquent une différence significative parmi les médianes des deux groupes avec un niveau de confiance de 95%.

**2. Résultats**

2.1 Evaluation d'activité antitumorale de l'anticorps 3C23K en combinaisons avec le carboplatine

Il apparaît que l'anticorps 3C23K ou 3C23K présente une activité antitumorale significative comparée au témoin (une solution NaCl) et à l'anticorps LFB-R565 (ou LFB-R565) (Figures 1 et 2; tableaux 2, 3, 4 and 5).Par rapport au témoin, les ratios T/C 3C23K diminuaient de J14 à J36, à J18 le ratio T/C était de 33% et était constamment inférieur à 23% jusqu'à J36 (tableau 4). Par rapport à l'anticorps irrelevant LFB-R565, les ratios T/C diminuaient de J14 à J36, à J18 le ratio T/C était de 41% et était constamment inférieur à 26% jusqu'à J36 (tableau 4).

De plus, le traitement avec l'anticorps irrelevant LFB-R565 ne présente pas d'activité anti-tumorale par rapport au témoin (Figure 1 et 2; tableaux 2, 3, 4 et 5) puisque les ratios T/C n'étaient jamais inférieurs à 68% (tableau 4).

Le carboplatine présente aussi une activité antitumorale significative comparée au témoin ou à l'anticorps irrelevant LFB-R565 (Figure 1 et 2; tableaux 2, 3, 4 et 5). De J18 à J36, les ratios T/C vis-à-vis du témoin diminuaient progressivement et la valeur était de 40% à J36, indiquant une activité anti-tumorale (tableau 4). De façon similaire, lorsque comparé à un groupe traité avec l'anticorps irrelevant LFB-R565, le ratio T/C diminuait de J25 à J36 pour atteindre 35% à J36 (tableau 4).

Des résultats similaires à ceux du groupe traité par le carboplatine ont été obtenus avec la combinaison carboplatine + LFB-R565, comparé au groupe témoin. De J18 à J36, les ratios T/C ont diminué progressivement et, à J36, le rapport T/C était de 40%, démontrant une activité antitumorale (tableau 4).

Lorsque le groupe traité par carboplatine + LFB-R565 était considéré comme le groupe traité et comparé au LFB-R565 seul, une diminution des ratios T/C a été observé à partir de D22 et deux rapports T/C inférieurs à 42% ont été trouvés, à J25 et J36 (tableau 4).

De plus, il n'y avait pas de différence entre les groupes traités par carboplatine + LFB-R565 ou par carboplatine seul (Figure 1; tableaux 2, 3, 4 et 5).

Le 3C23K (10mg/kg, Q3-4D12) et le carboplatine (60mg/kg, Q7D4) utilisés en monothérapie ont montré un effet antitumoral dans ce modèle de tumeur Cov434-AMHRII Asc1a5 xénogreffée et aux doses testées (Figure 1 and 2; tableaux 2, 3, 4 et 5).

De plus, dans les conditions testées, 3C23K a montré un effet antitumoral supérieur au carboplatine. En effet, le ratio T/C du groupe traité par 3C23K par rapport au groupe traité par le carboplatine a diminué de J14 à J39, et de J22 à J39, les ratios T/C étaient inférieurs à 42%, ce qui indique une activité antitumorale de 3C23K supérieure à celle du carboplatine (Tableau 4).

Quand le groupe traité par le carboplatine associé à 3C23K a été comparé à un groupe témoin, la combinaison a montré un effet antitumoral très fort : à J18 le ratio T/C était de 24% et de J22 à J36 les ratios T/C étaient inférieurs à 10% (Figure 1 et 2; Tableaux 2, 3, 4 and 5).

De plus, la combinaison du carboplatine (60mg/kg, Q7D4) et de 3C23K (10mg/kg, Q3-4D12) a montré un effet antitumoral plus fort que chacun des composants, 3C23K (10mg/kg, Q3-4D12) ou carboplatine (60mg/kg, Q7D4) utilisé en monothérapie (Figure 1 et 2; Tableaux 2, 3, 4 et 5).

En effet, quand le groupe traité par carboplatine + 3C23K a été comparé avec le groupe traité par le carboplatine, l'association a montré une plus grande activité antitumorale: à J14 le ratio T/C a commencé à diminuer et de J25 à J43 le ratio calculé était inférieur à 11%.

Par ailleurs, quand le groupe traité par 3C23K et carboplatine a été comparé au groupe traité par 3C23K, la combinaison a montré une activité antitumorale supérieure à celle de la monothérapie avec 3C23K. A J18, le ratio T/C a commencé à diminuer, il a été trouvé inférieur à 42% entre J22 et J29, ce qui indique un avantage antitumoral et a été trouvé inférieur à 11% entre J32 et J49, ce qui indique un grand avantage antitumoral (Figure 1 et 2; Tableaux 2, 3, 4 et 5).

De plus, le groupe traité par le carboplatine et 3C23K a montré également un avantage antitumoral comparé au carboplatine associé à un anticorps irrelevant LFB-R565 (Tableau 4).

Les traitements n'ont pas eu d'effet jusqu'au jour 32 (tableaux 6 et 7). A J32, une diminution transitoire du poids corporel a été observée pour les groupes traités avec carboplatine, carboplatine + LFB-R565 et carboplatine + 3C23K. Cette diminution, après la troisième injection de carboplatine, à J28, n'était pas supérieure à 15%, par comparaison avec les mesures précédentes à J25. La diminution était similaire pour les groupes traités avec carboplatine ou carboplatine + LFB-R565 (environ 15%) et était d'environ 1% pour le groupe traité par carboplatine + 3C23K. Dans ces trois groupes, la diminution de poids, légère et transitoire, n'a pas été considérée comme un effet toxique car elle n'a pas été confirmée après la 4ème injection de carboplatine, à J34 (tableaux 6 et 7).

## 3. Conclusions

Dans la présente étude les inventeurs ont évalué l'association de 3C23K à10mg/kg, Q3-4D12, et la dose sous-optimale de carboplatine 60mg/kg, Q7D4, sur des modèles de souris nude femelle xénogreffées par la tumeur Cov434-AMHRII Asc1a5, comparé à un groupe témoin. Un anticorps irrelevant, LFB-R565 (10mg/kg, Q3-4D12), a aussi été testé, seul ou en association avec le carboplatine.

Les résultats démontrent que le carboplatine (60mg/kg Q7D4) exerce une activité antitumorale sur Cov434-AMHRII Asc1a5. Les résultats démontrent aussi que le 3C23K (10mg/kg Q3-4D12) exerce une activité antitumorale sur Cov434-AMHRII Asc1a5.

Cependant, l'activité antitumorale observée avec le carboplatine seul était plus faible que celle observée avec le 3C23K seul (10mg/kg, Q3-4D12).

La combinaison du 3C23K et du carboplatine a été démontrée comme présentant une avantage lorsque comparée au 3C23K ou au carboplatine seuls et fourni a minima un effet additif.

Finalement, l'activité antitumorale de 3C23K seul ou en association avec le carboplatine est spécifique, car aucune efficacité n'a été observée avec un anticorps irrelevant, qu'il soit seul ou en combinaison.

## Tableau 1 : Résumé du schéma de traitement

| Schéma de traitement | | | | | | | | | |
|---|---|---|---|---|---|---|---|---|---|
| Date | Produit | témoinvéhicule | LFB-R565 | 3C23K | Carboplatine ou carboplatine | carboplatine+LFB-R565 | | carboplatine + 3C23K | |
| 19-sept | D11 | Randomisation des animaux | | | | | | | |

| | | | | | | | | | |
|---|---|---|---|---|---|---|---|---|---|
| 20-sept | D12 | | | | | | | | |
| 21-sept | D13 | TT | TT | TT | | | T<br>T | | T<br>T |
| 22-sept | D14 | | | | TT | TT | | TT | |
| 23-sept | D15 | TT | TT | TT | | | T<br>T | | T<br>T |
| 24-sept | D16 | | | | | | | | |
| 25-sept | D17 | | | | | | | | |
| 26-sept | D18 | TT | TT | TT | | | T<br>T | | T<br>T |
| 27-sept | D19 | | | | | | | | |
| 28-sept | D20 | | | | | | | | |
| 29-sept | D21 | | | | TT | TT | | TT | |
| 30-sept | D22 | TT | TT | TT | | | T<br>T | | T<br>T |
| 01-oct | D23 | | | | | | | | |
| 02-oct | D24 | | | | | | | | |
| 03-oct | D25 | TT | TT | TT | | | T<br>T | | T<br>T |
| 04-oct | D26 | | | | | | | | |
| 05-oct | D27 | | | | | | | | |
| 06-oct | D28 | | | | TT | TT | | TT | |
| 07-oct | D29 | TT | TT | TT | | | T<br>T | | T<br>T |
| 08-oct | D30 | | | | | | | | |
| 09-oct | D31 | | | | | | | | |
| 10-oct | D32 | TT | TT | TT | | | T<br>T | | T<br>T |
| 11-oct | D33 | | | | | | | | |
| 12-oct | D34 | | | | | | | | |
| 13-oct | D35 | | | | TT | TT | | TT | |
| 14-oct | D36 | TT | TT | TT | | | T<br>T | | T<br>T |
| 15-oct | D37 | | | | | | | | |
| 16-oct | D38 | | | | | | | | |
| 17-oct | D39 | TT | TT | TT | | | T<br>T | | T<br>T |
| 18-oct | D40 | | | | | | | | |
| 19-oct | D41 | | | | | | | | |
| 20-oct | D42 | | | | | | | | |
| 21-oct | D43 | TT | TT | TT | | | T<br>T | | T<br>T |
| 22-oct | D44 | | | | | | | | |

| | | | | | | | | | | |
|---|---|---|---|---|---|---|---|---|---|---|
| **23-oct** | **D45** | | | | | | | | | |
| **24-oct** | **D46** | TT | TT | TT | | | TT | | TT |
| **25-oct** | **D47** | | | | | | | | | |
| **26-oct** | **D48** | | | | | | | | | |
| **27-oct** | **D49** | | | | | | | | | |

**Tableau 2 : Résumé des données brutes de volume tumoral individuel**

| Groupe | Numéro de souris | Volume tumoral individuel | | | | | | | | | | | |
|---|---|---|---|---|---|---|---|---|---|---|---|---|---|
| | | 9/19/11 | 9/22/11 | 9/26/11 | 9/30/11 | 10/3/11 | 10/7/11 | 10/10/11 | 10/14/11 | 10/17/11 | 10/21/11 | 10/24/11 | 10/27/11 |
| | | J11 | J14 | J18 | J22 | J25 | J29 | J32 | J36 | J39 | J43 | J46 | J49 |
| NaCl | 1-SM | 91 | 126 | 250 | 504 | 819 | 952 | 1020 | 2404 | | | | |
| | 1-OD | 72 | 169 | 143 | 297 | 303 | 1320 | 891 | 1260 | 1848 | 1564 | 2622 | |
| | 1-OG | 96 | 147 | 216 | 520 | 700 | 588 | 2993 | | | | | |
| | 1-2O | 107 | 180 | 240 | 468 | 480 | 644 | 864 | 1540 | 1560 | 2625 | | |
| | 1-2OD | 99 | 192 | 144 | 216 | 330 | 462 | 660 | 1144 | 2280 | | | |
| | 2-SM | 60 | 163 | 248 | 252 | 336 | 504 | 1045 | 1617 | 2070 | | | |
| | 2-OD | 74 | 120 | 135 | 392 | 441 | 936 | 1053 | 1589 | 2024 | | | |
| | 2-OG | 158 | 234 | 324 | 672 | 495 | 819 | 1181 | 1607 | 1386 | 2178 | | |
| | 2-20 | 77 | 123 | 108 | 180 | 264 | 420 | 490 | 630 | 828 | 912 | 1485 | 2080 |
| LFB-R565 | 3-SM | 90 | 203 | 173 | 378 | 432 | 792 | 1287 | 2321 | | | | |
| | 3-OD | 72 | 110 | 140 | 126 | 216 | 792 | 356 | 588 | 720 | 995 | 1215 | 1672 |
| | 3-OG | 96 | 154 | 297 | 360 | 465 | 840 | 1140 | 2166 | 2166 | | | |
| | 3-2O | 153 | 175 | 460 | 324 | 520 | 588 | 690 | 1824 | 1790 | 2112 | | |
| | 3-2OD | 108 | 154 | 192 | 363 | 504 | 980 | 1536 | 2052 | | | | |
| | 4-SM | 58 | 176 | 216 | 270 | 385 | 585 | 1122 | 1980 | 2576 | | | |
| | 4-OD | 72 | 110 | 140 | 197 | 410 | 693 | 616 | 774 | 528 | 624 | 413 | 630 |
| | 4-OG | 81 | 95 | 108 | 216 | 297 | 504 | 569 | 1584 | 1454 | 2079 | | |
| | 4-2O | 60 | 95 | 160 | 270 | 288 | 504 | 660 | 1144 | 1073 | 1672 | 1785 | 2321 |

(suite)

| | | Volume tumoral individuel | | | | | | | | | | | |
|---|---|---|---|---|---|---|---|---|---|---|---|---|---|
| Groupe | Numéro de souris | 9/19/11 | 9/22/11 | 9/26/11 | 9/30/11 | 10/3/11 | 10/7/11 | 10/10/11 | 10/14/11 | 10/17/11 | 10/21/11 | 10/24/11 | 10/27/11 |
| | | J11 | J14 | J18 | J22 | J25 | J29 | J32 | J36 | J39 | J43 | J46 | J49 |
| 3C23K | 5-SM | 98 | 104 | 72 | 60 | 0 | 0 | 0 | 0 | 0 | 0 | 0 | 0 |
| | 5-OD | 90 | 179 | 88 | 150 | 126 | 322 | 501 | 653 | 784 | 1171 | 1683 | 1870 |
| | 5-OG | 61 | 60 | 36 | 30 | 0 | 27 | 27 | 87 | 210 | 420 | 598 | 1188 |
| | 5-2O | 80 | 72 | 21 | 0 | 0 | 0 | 0 | 0 | 0 | 0 | 0 | 0 |
| | 5-2OD | 57 | 67 | 41 | 36 | 24 | 48 | 15 | 0 | 0 | 0 | 0 | 0 |
| | 6-SM | 147 | 216 | 140 | 131 | 180 | 225 | 275 | 363 | 315 | 631 | 504 | 784 |
| | 6-OD | 113 | 166 | 80 | 77 | 74 | 146 | 180 | 182 | 182 | 393 | 275 | 655 |
| | 6-OG | 72 | 84 | 72 | 158 | 216 | 385 | 644 | 784 | 1200 | 1403 | 1650 | 2646 |
| | 6-2O | 72 | 53 | 53 | 70 | 88 | 210 | 350 | 432 | 546 | 731 | 518 | 945 |
| carboplatine | 7-SM | 53 | 104 | 98 | 378 | 140 | 270 | 351 | 630 | 660 | 864 | 1071 | 1568 |
| | 7-OD | 142 | 210 | 173 | 126 | 240 | 200 | 243 | 424 | 484 | 592 | 501 | 819 |
| | 7-OG | 61 | 162 | 112 | 360 | 270 | 360 | 578 | 1148 | 1134 | 1881 | 2205 | |
| | 7-2O | 102 | 210 | 336 | 324 | 616 | 768 | 864 | 1610 | 1495 | 2112 | | |
| | 7-2OD | 90 | 126 | 248 | 363 | 152 | 360 | 653 | 893 | 1488 | 2038 | | |
| | 8-SM | 72 | 123 | 140 | 270 | 358 | 363 | 504 | 462 | 447 | 882 | 810 | 1125 |
| | 8-OD | 81 | 75 | 90 | 156 | 98 | 168 | 220 | 308 | 347 | 438 | 378 | 480 |
| | 8-OG | 70 | 134 | 112 | 198 | 243 | 420 | 462 | 774 | 910 | 1105 | 1450 | 1650 |
| | 8-2O | 114 | 168 | 90 | 168 | 210 | 187 | 275 | 594 | 423 | 672 | 878 | 980 |

(suite)

| Groupe | Numéro de souris | Volume tumoral individuel | | | | | | | | | | | |
|---|---|---|---|---|---|---|---|---|---|---|---|---|---|
| | | 9/19/11 | 9/22/11 | 9/26/11 | 9/30/11 | 10/3/11 | 10/7/11 | 10/10/11 | 10/14/11 | 10/17/11 | 10/21/11 | 10/24/11 | 10/27/11 |
| | | J11 | J14 | J18 | J22 | J25 | J29 | J32 | J36 | J39 | J43 | J46 | J49 |
| LFB-R565 + carboplatine | 9-SM | 116 | 203 | 216 | 297 | 298 | 420 | 518 | 720 | 936 | 1323 | 1440 | 2112 |
| | 9-OD | 72 | 90 | 123 | 126 | 112 | 180 | 385 | 594 | 608 | 900 | 965 | 1233 |
| | 9-OG | 63 | 90 | 126 | 112 | 91 | 144 | 180 | 350 | 501 | 429 | 592 | 686 |
| | 9-2O | 102 | 150 | 180 | 220 | 170 | 330 | 336 | 726 | 564 | 675 | 570 | 936 |
| | 9-2OD | 98 | 168 | 299 | 420 | 432 | 1040 | 1134 | 2288 | | | | |
| | 10-SM | 51 | 210 | 193 | 210 | 264 | 410 | 455 | 704 | 774 | 1008 | 1378 | 1881 |
| | 10-OD | 130 | 180 | 338 | 280 | 193 | 865 | 798 | 1232 | 1449 | 1783 | 1320 | 2185 |
| | 10-OG | 68 | 72 | 85 | 135 | 123 | 192 | 266 | 462 | 840 | 1081 | 995 | 995 |
| | 10-2O | 81 | 90 | 98 | 108 | 117 | 210 | 248 | 216 | 378 | 293 | | |
| 3C23K + carboplatine | 11-SM | 63 | 168 | 140 | 120 | 105 | 96 | 88 | 70 | 45 | 165 | 175 | 189 |
| | 11-OD | 123 | 102 | 54 | 38 | 24 | 40 | 18 | 0 | 24 | 66 | 69 | 90 |
| | 11-OG | 83 | 95 | 45 | 24 | 23 | 35 | 45 | 48 | 50 | | | |
| | 11-2O | 83 | 96 | 53 | 24 | 18 | 81 | 18 | 18 | 0 | 18 | 23 | 24 |
| | 11-2OD | 123 | 112 | 112 | 24 | 0 | 0 | 0 | 18 | 0 | 18 | 34 | 81 |
| | 12-SM | 50 | 84 | 18 | 0 | 0 | 0 | 0 | 18 | 11 | 24 | 23 | 28 |
| | 12-OD | 72 | 77 | 47 | 72 | 54 | 108 | 88 | 140 | 100 | 242 | 250 | 325 |
| | 12-OG | 107 | 158 | 21 | 0 | 0 | 32 | 0 | 0 | 0 | 24 | 42 | 45 |
| | 12-2O | 65 | 60 | 72 | 98 | 132 | 112 | 105 | 160 | 184 | 357 | 273 | 539 |

**Tableau 3 : Résumé des données brutes de volume tumoral moyen et de la déviation standard (SD)**

| Volume tumoral moyen | | | | | | | | | | | | | |
|---|---|---|---|---|---|---|---|---|---|---|---|---|---|
| Groupe | Jour | 11 | 14 | 18 | 22 | 25 | 29 | 32 | 36 | 39 | 43 | 46 | 49 |
| Témoin | Moyenne | 93 | 161 | 201 | 389 | 463 | 738 | 1133 | 1474 | | | | |
| | SD | 10 | 13 | 25 | 58 | 67 | 104 | 258 | 191 | | | | |
| LFB-R565 | Moyenne | 10 | 14 | 38 | 31 | 37 | 58 | 140 | 222 | | | | |
| | SD | 10 | 21 | 12 | 20 | 29 | 51 | 84 | 105 | 146 | 181 | 233 | 321 |
| 3C23K | Moyenne | 88 | 111 | 67 | 79 | 79 | 151 | 221 | 278 | 360 | 528 | 581 | 899 |
| | SD | 10 | 21 | 12 | 20 | 29 | 51 | 84 | 105 | 146 | 181 | 233 | 321 |
| carboplatine | Moyenne | 87 | 146 | 155 | 260 | 258 | 344 | 461 | 760 | 821 | 1176 | | |
| | SD | 10 | 16 | 30 | 35 | 55 | 65 | 76 | 145 | 161 | 232 | | |
| LFB-R565 + carboplatine | Moyenne | 87 | 139 | 184 | 212 | 200 | 421 | 480 | 810 | 756 | 936 | | |
| | SD | 9 | 19 | 31 | 37 | 40 | 113 | 108 | 221 | 127 | 183 | | |
| 3C23K + carboplatine | Moyenne | 85 | 106 | 62 | 44 | 40 | 56 | 40 | 53 | 46 | 114 | 111 | 165 |
| | SD | 9 | 12 | 13 | 14 | 16 | 15 | 14 | 20 | 21 | 46 | 38 | 65 |

**Tableau 4 : Données brutes des volumes tumoraux médians et des rapports T/C**

| Volume tumoral moyen | | | | | | | | | | | | |
|---|---|---|---|---|---|---|---|---|---|---|---|---|
| Jour | 11 | 14 | 18 | 22 | 25 | 29 | 32 | 36 | 39 | 43 | 46 | 49 |
| Témoin | 91 | 163 | 216 | 392 | 441 | 644 | 1020 | 1564 | | | | |
| LFB-R565 | 81 | 154 | 173 | 270 | 410 | 693 | 690 | 1824 | | | | |
| 3C23K | 80 | 84 | 72 | 70 | 74 | 146 | 180 | 182 | 210 | 420 | 504 | 784 |
| carboplatine | 81 | 134 | 112 | 270 | 240 | 360 | 462 | 630 | 660 | 882 | | |
| LFB-R565 + carboplatine | 81 | 150 | 180 | 210 | 170 | 330 | 385 | 704 | 691 | 954 | | |
| 3C23K + carboplatine | 83 | 96 | 53 | 24 | 23 | 40 | 18 | 18 | 24 | 45 | 55 | 86 |
| Ratio TC (%) | | | | | | | | | | | | |
| Témoin - LFB-R565 | 89 | 95 | 80 | 69 | 93 | 108 | 68 | 117 | | | | |
| Témoin - 3C23K | 88 | 52 | 33 | 18 | 17 | 23 | 18 | 12 | | | | |
| Témoin - carboplatine | 89 | 82 | 52 | 69 | 54 | 56 | 45 | 40 | | | | |
| Témoin - LFB-R565 + carboplatine | 89 | 92 | 83 | 54 | 39 | 51 | 38 | 45 | | | | |
| Témoin - 3C23K + carboplatine | 91 | 59 | 24 | 6 | 5 | 6 | 2 | 1 | | | | |
| LFB-R565 - 3C23K | 99 | 55 | 41 | 26 | 18 | 21 | 26 | 10 | | | | |
| LFB-R565 - carboplatine | 100 | 87 | 65 | 100 | 59 | 52 | 67 | 35 | | | | |
| LFB-R565 - LFB-R565 + carboplatine | 100 | 97 | 104 | 78 | 42 | 48 | 56 | 39 | | | | |
| LFB-R565 - 3C23K + carboplatine | 102 | 63 | 30 | 9 | 5 | 6 | 3 | 1 | | | | |

(suite)

| Ratio TC (%) | | | | | | | | | | | | |
|---|---|---|---|---|---|---|---|---|---|---|---|---|
| carboplatine - 3C23K | 99 | 63 | 64 | 26 | 31 | 41 | 39 | 29 | 32 | 48 | | |
| carboplatine - LFB-R565 + carboplatine | 100 | 112 | 161 | 78 | 71 | 92 | 83 | 112 | 105 | 108 | | |
| carboplatine - 3C23K + carboplatine | 102 | 72 | 47 | 9 | 9 | 11 | 4 | 3 | 4 | 5 | | |
| 3C23K - 3C23K + carboplatine | 103 | 115 | 73 | 34 | 31 | 27 | 10 | 10 | 11 | 11 | 11 | 11 |
| LFB-R565 + carboplatine - 3C23K + carboplatine | 102 | 64 | 29 | 11 | 13 | 12 | 5 | 3 | 3 | 5 | | |

**Tableau 5 : Analyse statistique des volumes tumoraux**

| Analyse statistique des volumes tumoraux | | | | | | | |
|---|---|---|---|---|---|---|---|
| Jour | Groupes | Ratio F | Valeur P | Sign | Test statistic | Valeur P | Sign |
| | Témoin - LFB-R565 | 0,13 | 0,73 | | 0,44 | 0,51 | |
| | Témoin - 3C23K | 0,13 | 0,72 | | 0,33 | 0,57 | |
| | Témoin - carboplatine | 0,16 | 0,69 | | 0,28 | 0,60 | |
| | Témoin - LFB-R565 + carboplatine | 0,21 | 0,66 | | 0,12 | 0,72 | |
| | Témoin - 3C23K + carboplatine | 0,31 | 0,59 | | 0,24 | 0,63 | |
| | LFB-R565 - 3C23K | 0,00 | 1,00 | | 0,00 | 1,00 | |
| | LFB-R565 - carboplatine | 0,00 | 0,97 | | 0,00 | 0,96 | |
| | LFB-R565 - LFB-R565 + carboplatine | 0,01 | 0,94 | | 0,01 | 0,93 | |
| | LFB-R565 - 3C23K + carboplatine | 0,03 | 0,86 | | 0,00 | 0,96 | |
| | carboplatine - 3C23K | 0,00 | 0,97 | | 0,00 | 0,96 | |
| | carboplatine - LFB-R565 + carboplatine | 0,00 | 0,97 | | 0,00 | 1,00 | |
| | carboplatine - 3C23K + carboplatine | 0,02 | 0,89 | | 0,00 | 1,00 | |
| | 3C23K - LFB-R565 + carboplatine | 0,01 | 0,94 | | 0,01 | 0,93 | |
| | 3C23K - 3C23K + carboplatine | 0,03 | 0,86 | | 0,00 | 0,96 | |
| J11 | LFB-R565 + carboplatine - 3C23K + carboplatine | 0,01 | 0,92 | | 0,00 | 0,96 | |
| | Témoin - LFB-R565 | 1,23 | 0,28 | | 1,22 | 0,27 | |
| | Témoin - 3C23K | 4,54 | 0,05 | * | 3,60 | 0,06 | |
| | Témoin - carboplatine | 0,64 | 0,44 | | 0,57 | 0,45 | |
| | Témoin - LFB-R565 + carboplatine | 1,03 | 0,32 | | 0,63 | 0,43 | |
| | Témoin - 3C23K + carboplatine | 10,41 | 0,01 | * | 7,74 | 0,01 | * |
| | LFB-R565 - 3C23K | 1,57 | 0,23 | | 1,88 | 0,17 | |
| | LFB-R565 - carboplatine | 0,05 | 0,83 | | 0,10 | 0,76 | |
| | LFB-R565 - LFB-R565 + carboplatine | 0,01 | 0,93 | | 0,20 | 0,66 | |
| | LFB-R565 - 3C23K + carboplatine | 3,96 | 0,06 | | 2,98 | 0,08 | |
| | carboplatine - 3C23K | 1,88 | 0,19 | | 2,00 | 0,16 | |

(suite)

| Analyse statistique des volumes tumoraux | | | | | | | |
|---|---|---|---|---|---|---|---|
| Jour | Groupes | Ratio F | Valeur P | Sign | Test statistic | Valeur P | Sign |
| | carboplatine - LFB-R565 + carboplatine | 0,08 | 0,79 | | 0,13 | 0,72 | |
| | carboplatine - 3C23K + carboplatine | 4,26 | 0,06 | | 3,78 | 0,05 | * |
| | 3C23K - LFB-R565 + carboplatine | 1,08 | 0,31 | | 1,76 | 0,18 | |
| | 3C23K - 3C23K + carboplatine | 0,05 | 0,82 | | 0,10 | 0,76 | |
| J14 | LFB-R565 + carboplatine - 3C23K + carboplatine | 2,39 | 0,14 | | 1,13 | 0,29 | |
| | Témoin - LFB-R565 | 0,04 | 0,84 | | 0,05 | 0,83 | |
| | Témoin - 3C23K | 25,29 | 0,00 | * | 11,57 | 0,00 | * |
| | Témoin - carboplatine | 1,51 | 0,24 | | 2,26 | 0,13 | |
| | Témoin - LFB-R565 + carboplatine | 0,19 | 0,67 | | 0,63 | 0,43 | |
| | Témoin - 3C23K + carboplatine | 25,53 | 0,00 | * | 10,96 | 0,00 | * |
| | LFB-R565 - 3C23K | 14,01 | 0,00 | * | 11,62 | 0,00 | * |
| | LFB-R565 - carboplatine | 1,39 | 0,26 | | 2,27 | 0,13 | |
| | LFB-R565 - LFB-R565 + carboplatine | 0,29 | 0,59 | | 0,20 | 0,66 | |
| | LFB-R565 - 3C23K + carboplatine | 14,50 | 0,00 | * | 11,01 | 0,00 | * |
| | carboplatine - 3C23K | 8,37 | 0,01 | * | 9,59 | 0,00 | * |
| | carboplatine - LFB-R565 + carboplatine | 0,50 | 0,49 | | 0,78 | 0,38 | |
| | carboplatine - 3C23K + carboplatine | 8,86 | 0,01 | * | 7,54 | 0,01 | * |
| | 3C23K - LFB-R565 + carboplatine | 13,69 | 0,00 | * | 9,84 | 0,00 | * |
| | 3C23K - 3C23K + carboplatine | 0,07 | 0,80 | | 0,20 | 0,66 | |
| J18 | LFB-R565 + carboplatine - 3C23K + carboplatine | 14,18 | 0,00 | * | 9,28 | 0,00 | * |
| | Témoin - LFB-R565 | 3,19 | 0,09 | | 2,00 | 0,16 | |
| | Témoin - 3C23K | 28,51 | 0,00 | * | 12,79 | 0,00 | * |
| | Témoin - carboplatine | 4,01 | 0,06 | | 3,28 | 0,07 | |
| | Témoin - LFB-R565 + carboplatine | 7,35 | 0,02 | * | 5,28 | 0,02 | * |
| | Témoin - 3C23K + carboplatine | 36,82 | 0,00 | * | 12,86 | 0,00 | * |
| | LFB-R565 - 3C23K | 33,73 | 0,00 | * | 10,98 | 0,00 | * |
| | LFB-R565 - carboplatine | 0,17 | 0,69 | | 0,13 | 0,72 | |
| | LFB-R565 - LFB-R565 + carboplatine | 2,12 | 0,16 | | 1,76 | 0,18 | |
| | LFB-R565 - 3C23K + carboplatine | 52,97 | 0,00 | * | 12,87 | 0,00 | * |
| | carboplatine - 3C23K | 22,58 | 0,00 | * | 10,39 | 0,00 | * |
| | carboplatine - LFB-R565 + carboplatine | 0,99 | 0,33 | | 1,12 | 0,29 | |
| | carboplatine - 3C23K + carboplatine | 35,55 | 0,00 | * | 12,86 | 0,00 | * |
| | 3C23K - LFB-R565 + carboplatine | 11,13 | 0,00 | * | 6,79 | 0,01 | * |
| | 3C23K - 3C23K + carboplatine | 2,19 | 0,16 | | 2,14 | 0,14 | |
| J22 | LFB-R565 + carboplatine - 3C23K + carboplatine | 19,45 | 0,00 | * | 11,62 | 0,00 | * |
| | Témoin - LFB-R565 | 1,02 | 0,33 | | 0,44 | 0,51 | |
| | Témoin - 3C23K | 31,60 | 0,00 | * | 12,84 | 0,00 | * |

(suite)

| Analyse statistique des volumes tumoraux | | | | | | | |
|---|---|---|---|---|---|---|---|
| Jour | Groupes | Ratio F | Valeur P | Sign | Test statistic | Valeur P | Sign |
| | Témoin - carboplatine | 6,33 | 0,02 | * | 6,33 | 0,01 | * |
| | Témoin - LFB-R565 + carboplatine | 12,96 | 0,00 | * | 9,56 | 0,00 | * |
| | Témoin - 3C23K + carboplatine | 42,71 | 0,00 | * | 12,84 | 0,00 | * |
| | LFB-R565 - 3C23K | 50,07 | 0,00 | * | 12,54 | 0,00 | * |
| | LFB-R565 - carboplatine | 4,51 | 0,05 | * | 5,07 | 0,02 | * |
| | LFB-R565 - LFB-R565 + carboplatine | 13,94 | 0,00 | * | 7,50 | 0,01 | * |
| | LFB-R565 - 3C23K + carboplatine | 83,99 | 0,00 | * | 12,84 | 0,00 | * |
| | carboplatine - 3C23K | 9,51 | 0,01 | * | 8,27 | 0,00 | * |
| | carboplatine - LFB-R565 + carboplatine | 0,84 | 0,37 | | 0,86 | 0,35 | |
| | carboplatine - 3C23K + carboplatine | 16,39 | 0,00 | * | 11,61 | 0,00 | * |
| | 3C23K - LFB-R565 + carboplatine | 6,89 | 0,02 | * | 5,09 | 0,02 | * |
| | 3C23K - 3C23K + carboplatine | 1,54 | 0,23 | | 0,73 | 0,39 | |
| J25 | LFB-R565 + carboplatine - 3C23K + carboplatine | 15,49 | 0,00 | * | 9,87 | 0,00 | * |
| | Témoin - LFB-R565 | 0,13 | 0,72 | | 0,00 | 1,00 | |
| | Témoin - 3C23K | 28,90 | 0,00 | * | 12,80 | 0,00 | * |
| | Témoin - carboplatine | 11,64 | 0,00 | * | 9,57 | 0,00 | * |
| | Témoin - LFB-R565 + carboplatine | 4,78 | 0,04 | * | 5,28 | 0,02 | * |
| | Témoin - 3C23K + carboplatine | 47,26 | 0,00 | * | 12,80 | 0,00 | * |
| | LFB-R565 - 3C23K | 56,20 | 0,00 | * | 12,83 | 0,00 | * |
| | LFB-R565 - carboplatine | 18,53 | 0,00 | * | 9,86 | 0,00 | * |
| | LFB-R565 - LFB-R565 + carboplatine | 5,30 | 0,04 | * | 4,31 | 0,04 | * |
| | LFB-R565 - 3C23K + carboplatine | 127,11 | 0,00 | * | 12,83 | 0,00 | * |
| | carboplatine - 3C23K | 6,17 | 0,02 | * | 4,31 | 0,04 | * |
| | carboplatine - LFB-R565 + carboplatine | 0,39 | 0,54 | | 0,03 | 0,86 | |
| | carboplatine - 3C23K + carboplatine | 21,04 | 0,00 | * | 12,82 | 0,00 | * |
| | 3C23K - LFB-R565 + carboplatine | 5,32 | 0,03 | * | 4,13 | 0,04 | * |
| | 3C23K - 3C23K + carboplatine | 3,64 | 0,07 | | 1,44 | 0,23 | |
| J29 | LFB-R565 + carboplatine - 3C23K + carboplatine | 11,49 | 0,00 | * | 12,80 | 0,00 | * |
| | Témoin - LFB-R565 | 0,80 | 0,39 | | 0,20 | 0,66 | |
| | Témoin - 3C23K | 12,70 | 0,00 | * | 11,57 | 0,00 | * |
| | Témoin - carboplatine | 7,03 | 0,02 | * | 9,56 | 0,00 | * |
| | Témoin - LFB-R565 + carboplatine | 6,13 | 0,02 | * | 7,25 | 0,01 | * |
| | Témoin - 3C23K + carboplatine | 20,13 | 0,00 | * | 12,87 | 0,00 | * |
| | LFB-R565 - 3C23K | 18,68 | 0,00 | * | 10,40 | 0,00 | * |
| | LFB-R565 - carboplatine | 8,06 | 0,01 | * | 6,33 | 0,01 | * |
| | LFB-R565 - LFB-R565 + carboplatine | 5,95 | 0,03 | * | 5,48 | 0,02 | * |
| | LFB-R565 - 3C23K + carboplatine | 40,84 | 0,00 | * | 12,87 | 0,00 | * |

(suite)

| Analyse statistique des volumes tumoraux | | | | | | | |
|---|---|---|---|---|---|---|---|
| Jour | Groupes | Ratio F | Valeur P | Sign | Test statistic | Valeur P | Sign |
| | carboplatine - 3C23K | 5,03 | 0,04 | * | 4,13 | 0,04 | * |
| | carboplatine - LFB-R565 + carboplatine | 0,02 | 0,88 | | 0,05 | 0,83 | |
| | carboplatine - 3C23K + carboplatine | 33,41 | 0,00 | * | 12,87 | 0,00 | * |
| | 3C23K - LFB-R565 + carboplatine | 3,99 | 0,06 | | 3,13 | 0,08 | * |
| | 3C23K - 3C23K + carboplatine | 5,01 | 0,04 | * | 1,92 | 0,17 | |
| J32 | LFB-R565 + carboplatine - 3C23K + carboplatine | 18,20 | 0,00 | * | 12,87 | 0,00 | * |
| | Témoin - LFB-R565 | 0,22 | 0,65 | | 0,28 | 0,60 | |
| | Témoin - 3C23K | 36,45 | 0,00 | * | 10,76 | 0,00 | * |
| | Témoin - carboplatine | 10,34 | 0,01 | * | 6,03 | 0,01 | * |
| | Témoin - LFB-R565 + carboplatine | 5,70 | 0,03 | * | 5,33 | 0,02 | * |
| | Témoin - 3C23K + carboplatine | 70,67 | 0,00 | * | 12,07 | 0,00 | * |
| | LFB-R565 - 3C23K | 32,80 | 0,00 | * | 11,01 | 0,00 | * |
| | LFB-R565 - carboplatine | 11,39 | 0,00 | * | 6,12 | 0,01 | * |
| | LFB-R565 - LFB-R565 + carboplatine | 7,22 | 0,02 | * | 5,07 | 0,02 | * |
| | LFB-R565 - 3C23K + carboplatine | 54,44 | 0,00 | * | 12,86 | 0,00 | * |
| | carboplatine - 3C23K | 8,19 | 0,01 | * | 5,50 | 0,02 | * |
| | carboplatine - LFB-R565 + carboplatine | 0,04 | 0,84 | | 0,02 | 0,89 | |
| | carboplatine - 3C23K + carboplatine | 26,34 | 0,00 | * | 12,86 | 0,00 | * |
| | 3C23K - LFB-R565 + carboplatine | 5,33 | 0,03 | * | 5,09 | 0,02 | * |
| | 3C23K - 3C23K + carboplatine | 4,99 | 0,04 | * | 1,68 | 0,19 | |
| J36 | LFB-R565 + carboplatine - 3C23K + carboplatine | 13,13 | 0,00 | * | 12,86 | 0,00 | * |
| | carboplatine - 3C23K | 5,05 | 0,04 | * | 4,70 | 0,03 | * |
| | carboplatine - LFB-R565 + carboplatine | 0,11 | 0,75 | | 0,00 | 1,00 | |
| | carboplatine - 3C23K + carboplatine | 25,47 | 0,00 | * | 12,84 | 0,00 | * |
| | 3C23K - LFB-R565 + carboplatine | 4,64 | 0,05 | * | 4,50 | 0,03 | * |
| | 3C23K - 3C23K + carboplatine | 5,08 | 0,04 | * | 2,34 | 0,13 | |
| J39 | LFB-R565 + carboplatine - 3C23K + carboplatine | 39,24 | 0,00 | * | 12,06 | 0,00 | * |
| | carboplatine - 3C23K | 5,46 | 0,03 | * | 4,32 | 0,04 | * |
| | carboplatine - LFB-R565 + carboplatine | 0,72 | 0,41 | | 0,33 | 0,56 | |
| | carboplatine - 3C23K + carboplatine | 20,12 | 0,00 | * | 12,03 | 0,00 | * |
| | 3C23K - LFB-R565 + carboplatine | 2,85 | 0,11 | | 2,38 | 0,12 | |
| | LFB-R565 + carboplatine - 3C23K + carboplatine | 21,66 | 0,00 | * | 10,63 | 0,00 | * |
| J43 | 3C23K - 3C23K + carboplatine | 4,91 | 0,04 | * | 1,34 | 0,25 | |
| J46 | 3C23K - 3C23K + carboplatine | 3,95 | 0,07 | | 1,34 | 0,25 | |
| J49 | 3C23K - 3C23K + carboplatine | 5,02 | 0,04 | * | 1,34 | 0,25 | |

**Tableau 6 : Résumé des données brutes de poids corporel individuel**

| Poids corporel individuel des souris | | | | | | | |
|---|---|---|---|---|---|---|---|
| Groupe | Numéro souris | 09/19/11 | 09/26/11 | 10/03/11 | 10/10/11 | 10/17/11 | 10/24/11 |
| | | J11 | J18 | J25 | J32 | J39 | J46 |
| NaCl | 1-SM | 25,6 | 27,3 | 28,2 | 29,2 | | |
| | 1-OD | 22,3 | 24,8 | 24,7 | 25,0 | 27,0 | 28,9 |
| | 1-OG | 25,0 | 27,6 | 28,0 | 29,0 | | |
| | 1-20 | 21,5 | 22,8 | 23,2 | 24,6 | 25,8 | |
| | 1-20D | 25,0 | 27,0 | 28,1 | 29,6 | 29,6 | |
| | 2-SM | 22,4 | 23,6 | 23,8 | 25,4 | 27,1 | |
| | 2-OD | 24,0 | 26,1 | 26,8 | 29,3 | 30,0 | |
| | 2-OG | 22,8 | 22,0 | 23,8 | 25,6 | 25,0 | |
| | 2-2O | 24,4 | 24,4 | 25,2 | 27,0 | 27,1 | 29,6 |
| LFB-R565 | 3-SM | 23,4 | 23,3 | 24,0 | 25,1 | | |
| | 3-OD | 22,2 | 23,6 | 23,8 | 23,9 | 25,2 | 27,0 |
| | 3-OG | 23,5 | 24,4 | 24,8 | 25,8 | | |
| | 3-2O | 21,5 | 21,8 | 22,3 | 23,5 | 24,6 | |
| | 3-2OD | 25,4 | 25,6 | 26,4 | 28,2 | | |
| | 4-SM | 23,6 | 25,0 | 25,1 | 26,6 | 28,2 | |
| | 4-OD | 27,1 | 26,4 | 28,2 | 29,7 | 30,5 | 30,7 |
| | 4-OG | 23,2 | 24,1 | 24,6 | 26,3 | 26,8 | |
| | 4-2O | 24,9 | 26,0 | 26,0 | 29,7 | 30,4 | 32,8 |
| 3C23K | 5-SM | 21,0 | 22,1 | 22,1 | 22,6 | 23,1 | 22,5 |
| | 5-OD | 20,2 | 21,8 | 22,2 | 23,5 | 24,1 | 24,9 |
| | 5-OG | 25,8 | 26,6 | 26,6 | 27,0 | 27,2 | 27,2 |
| | 5-2O | 24,9 | 25,2 | 25,0 | 25,2 | 27,8 | 26,0 |
| | 5-2OD | 23,5 | 26,4 | 26,0 | 26,0 | 26,5 | 27,2 |
| | 6-SM | 21,4 | 21,8 | 22,3 | 23,0 | 23,8 | 23,3 |
| | 6-OD | 23,0 | 24,2 | 25,0 | 24,0 | 26,0 | 25,7 |
| | 6-OG | 23,9 | 24,5 | 25,8 | 25,8 | 27,9 | 28,9 |
| | 6-2O | 24,9 | 24,0 | 24,3 | 24,8 | 25,3 | 25,8 |

(suite)

| Poids corporel individuel des souris | | | | | | | |
|---|---|---|---|---|---|---|---|
| Groupe | Numéro souris | 09/19/11 | 09/26/11 | 10/03/11 | 10/10/11 | 10/17/11 | 10/24/11 |
| | | J11 | J18 | J25 | J32 | J39 | J46 |
| carboplatine | 7-SM | 22,3 | 22,2 | 27,6 | 23,7 | 24,3 | 25,4 |
| | 7-OD | 25,8 | 24,6 | 28,3 | 24,5 | 23,9 | 25,7 |
| | 7-OG | 23,0 | 22,6 | 25,6 | 23,6 | 25,1 | 26,4 |
| | 7-2O | 27,1 | 26,1 | 32,7 | 28,0 | 27,2 | |
| | 7-2OD | 23,3 | 24,4 | 27,0 | 23,1 | 25,4 | |
| | 8-SM | 24,5 | 25,4 | 29,4 | 23,1 | 22,4 | 25,7 |
| | 8-OD | 22,7 | 23,6 | 25,6 | 21,1 | 20,0 | 21,7 |
| | 8-OG | 20,4 | 20,3 | 24,2 | 20,2 | 21,0 | 21,0 |
| | 8-2O | 22,3 | 22,0 | 26,0 | 23,6 | 23,3 | 25,0 |
| LFB-R565 + carboplatine | 9-SM | 22,2 | 21,8 | 26,2 | 20,9 | 22,7 | 23,1 |
| | 9-OD | 22,4 | 22,9 | 26,9 | 18,3 | 22,3 | 22,8 |
| | 9-OG | 24,0 | 23,9 | 27,7 | 23,0 | 23,8 | 24,8 |
| | 9-2O | 18,9 | 19,2 | 23,5 | 22,2 | 19,1 | 19,8 |
| | 9-2OD | 21,5 | 22,9 | 27,1 | 21,8 | | |
| | 10-SM | 23,0 | 22,6 | 23,9 | 22,7 | 25,3 | 24,3 |
| | 10-OD | 25,2 | 25,4 | 26,6 | 26,1 | 25,6 | 27,8 |
| | 10-OG | 25,8 | 25,3 | 28,2 | 27,0 | 26,2 | 27,5 |
| | 10-2O | 23,4 | 23,3 | 23,6 | 21,4 | 18,6 | |
| 3C23K + carboplatine | 11-SM | 26,8 | 26,8 | 26,3 | 27,0 | 26,9 | 27,5 |
| | 11-OD | 21,3 | 21,5 | 22,6 | 22,8 | 21,4 | 23,2 |
| | 11-OG | 20,0 | 19,2 | 20,1 | 18,8 | 17,0 | |
| | 11-2O | 24,0 | 24,8 | 23,1 | 24,0 | 23,1 | 25,0 |
| | 11-2OD | 24,6 | 23,5 | 22,9 | 25,2 | 22,5 | 24,0 |
| | 12-SM | 21,8 | 23,7 | 24,0 | 22,5 | 21,0 | 22,5 |
| | 12-OD | 22,3 | 20,5 | 23,7 | 22,7 | 22,3 | 23,7 |
| | 12-OG | 22,7 | 24,2 | 24,4 | 23,1 | 21,6 | 22,8 |
| | 12-2O | 22,8 | 21,9 | 25,0 | 23,6 | 23,6 | 25,2 |

**Tableau 7 : Résumé des données brutes de moyennes de poids corporel et de la déviation standard (SD).**

| Poids corporel moyen des souris | | | | | | | |
|---|---|---|---|---|---|---|---|
| Groupe | Jour | 11 | 18 | 25 | 32 | 39 | 46 |
| Témoin | Moy. | 23,7 | 25,1 | 25,8 | 27,2 | | |
| | SD | 1,5 | 2,0 | 2,0 | 2,1 | | |
| LFB-R565 | Moy. | 23,9 | 24,5 | 25,0 | 26,5 | | |
| | SD | 1,7 | 1,5 | 1,7 | 2,3 | | |

(suite)

| Poids corporel moyen des souris | | | | | | | |
|---|---|---|---|---|---|---|---|
| Groupe | Jour | 11 | 18 | 25 | 32 | 39 | 46 |
| 3C23K | Moy. | 23,2 | 24,1 | 24,4 | 24,7 | 25,7 | 25,7 |
| | SD | 1,9 | 1,9 | 1,8 | 1,5 | 1,8 | 2,0 |
| carboplatine | Moy. | 23,5 | 23,5 | 27,4 | 23,4 | 23,6 | |
| | SD | 2,0 | 1,8 | 2,5 | 2,2 | 2,2 | |
| LFB-R565 + carboplatine | Moy. | 22,9 | 23,0 | 26,0 | 22,6 | 23,0 | |
| | SD | 2,1 | 1,9 | 1,8 | 2,6 | 2,9 | |
| 3C23K + carboplatine | Moy. | 22,9 | 22,9 | 23,6 | 23,3 | 22,2 | 24,2 |
| | SD | 2,0 | 2,3 | 1,7 | 2,2 | 2,6 | 1,6 |

2.2 Evaluation d'activité antitumorale de l'anticorps 3C23K en combinaison avec le paclitaxel

L'anticorps 3C23K et le paclitaxel sont évalués *in vivo* en monothérapie ou en association dans les souris ayant reçu l'injection des cellules tumorales Cov434-AMHRII Asc1a5 (une lignée cellulaire de cancer ovarien humain). Le traitement est initié le jour 14 après l'injection.

Il a été observé que l'anticorps 3C23K administré à 10 mg/kg selon le régime Q7D4 décrit ci-dessus présente une activité antitumorale significative comparée à une solution témoin (une solution NaCl) (Figures 3 et 4; tableaux 9, 11, 12, 13 14 and 15).

Par rapport au groupe traité par la solution témoin, les ratios T/C du groupe traité par l'anticorps 3C23K diminuaient à partir de J20. A J20 le ratio T/C était de 57% et était constamment inférieur à 29% jusqu'à J40 (tableau 12).

Le paclitaxel seul ne montre qu'une inhibition modeste de la croissance de la tumeur comparée à la solution témoin. De J24 à J41, le ratio T/C varie de 83% à 53%.

Comparée au témoin, une plus importante activité antitumorale a été obtenue dans le groupe traité par la combinaison de l'anticorps 3C23K et du paclitaxel (Figures 3 et 4; tableaux 9, 11, 12, 13 14 and 15). Le ratio T/C était inférieur à 38% à J20 et était toujours inférieur à 28% jusqu'à J41 (tableau 12). En plus, le ratio T/C à J31, J35 et J41 était respectivement 6%, 11% et 12%. Comparés au résultat décrit par Bissery et al. en 1991, les résultats de la présente invention montrent que le ratio T/C était autour de et inférieur à 10% entre J31 et J41, cela indiquant que le traitement par une combinaison de l'anticorps 3C23K et du paclitaxel est une thérapie efficace.

De plus, l'anticorps 3C23K administré à 10 mg/kg selon le régime Q7D4 a montré un effet antitumoral supérieur à celui du paclitaxel. En effet, le ratio T/C du groupe traité par 3C23K par rapport au groupe traité par le paclitaxel a diminué à partir du J20. Le ratio T/C à J20 était 47% et inférieur à 39% à J41 (tableau 12), ce qui indique une activité antitumorale de 3C23K supérieure à celle du paclitaxel.

La combinaison de l'anticorps 3C23K (10mg/kg, Q7D4) et du paclitaxel (15mg/kg, le régime Q7D4) a montré un effet antitumoral plus fort que chacun des composants, 3C23K (10mg/kg, Q7D4) ou paclitaxel (15mg/kg, le régime Q7D4) utilisé en monothérapie.

En effet, quand le groupe traité par la susdite combinaison a été comparé avec le groupe traité par le paclitaxel, la combinaison a montré une plus grande activité antitumorale : à J20 le ratio T/C a commencé à diminuer et le ratio était 31% à J20 et inférieur à 35% jusqu'à J41 (tableau 12).

Quand le groupe traité par la susdite combinaison a été comparé avec le groupe traité par l'anticorps 3C23K, la combinaison a montré une activité antitumorale supérieure à celle de la monothérapie avec 3C23K. A J31, le ratio T/C a commencé à diminuer, Le ratio T/C était 27% à J31 et inférieur à 42% jusqu'à J48 (tableau 12). A J51, le ratio T/C n'est que 51%, cela indiquant néanmoins une différence entre ces deux groupes, car une inhibition de 49% de la croissance tumorale était observée (tableau 12).

L'analyse statistique du volume tumoral confirme les résultats d'analyse de ratio T/C.

L'anticorps 3C23K en monothérapie et l'anticorps 3C23K en association avec le paclitaxel montrent une activité antitumorale significative comparée à celle du témoin (une solution NaCl) à partir de J20 à J41 (tableau 13).

Le paclitaxel en monothérapie n'a pas d'activité significativement différente de celle du témoin (tableau 13).

L'anticorps 3C23K en monothérapie présente une activité antitumorale significativement différente de celle du paclitaxel en monothérapie à partir de J20 à J41 (tableau 13).

Comparée au traitement du paclitaxel en monothérapie, le traitement par la combinaison de l'anticorps 3C23K et du paclitaxel présente une meilleure activité antitumorale à partir de J20 à J41 (tableau 13).

L'analyse statistique sur le paramètre de survie confirme également les résultats de ratio T/C et d'analyse sur le volume tumoral.

L'anticorps 3C23K en monothérapie et la combinaison de l'anticorps 3C23K et du paclitaxel montrent respectivement une activité antitumorale significative comparée à celle du témoin (tableau 15).

Le paclitaxel en monothérapie n'a pas d'activité significativement différente de celle du témoin (tableau 15).

L'anticorps 3C23K en monothérapie présente une activité antitumorale significativement différente de celle du paclitaxel en monothérapie (tableau 15).Comparée au traitement du paclitaxel en monothérapie, le traitement par la combinaison de l'anticorps 3C23K et du paclitaxel présente une meilleure activité antitumorale (tableau 15).

Les traitements n'ont pas d'impact sur le poids corporel (tableau 8 et 10).

En conclusion, la combinaison de l'anticorps 3C23K et du paclitaxel présente un avantage par rapport au 3C23K ou au paclitaxel seuls et fourni a minima un effet additif.

Tableau 8 : Résumé des données brutes de poids corporel individuel

| Poids corporel individuel | | | | | | | | | |
|---|---|---|---|---|---|---|---|---|---|
| Group | Nombre de souris | D20 | D27 | D35 | D41 | D48 | D55 | D62 | D69 |
| Témoin | 1-SM | 23,4 | 24,1 | 26,0 | 26,9 | | | | |
| | 1-OD | 23,6 | 25,4 | 26,5 | | | | | |
| | 1-OG | 23,1 | 24,6 | 25,0 | 26,1 | | | | |
| | 1-2O | 21,8 | 23,4 | 25,2 | 25,4 | | | | |
| | 1-2OG | 23,7 | 25,8 | 26,8 | 27,8 | | | | |
| | 1-2OG/OD | 22,5 | 23,7 | 24,8 | 25,0 | | | | |
| | 10-2OD | 22,1 | 23,4 | 24,3 | 23,5 | 24,7 | 26,1 | | |
| | 10-2OG | 22,4 | 23,5 | 28,4 | 27,8 | 28,5 | 29,6 | | |
| 3C23K 10mg/kg q7d4 | 2-SM | 21,3 | 21,8 | 23,0 | 22,4 | 23,3 | 24,6 | | |
| | 2-OD | 25,0 | 24,8 | 26,8 | 27,0 | 27,5 | | | |
| | 2-OG | 24,8 | 26,3 | 27,0 | 27,6 | 27,2 | 27,5 | | |
| | 2-2O | 23,7 | 25,0 | 25,4 | 27,0 | 25,4 | 25,5 | 26,6 | 26,6 |
| | 2-2OD | 25,5 | 26,3 | 26,7 | 27,0 | 26,8 | 26,9 | 27,6 | 27,7 |
| | 2-2OG | 27,1 | 26,9 | 28,7 | 29,2 | 28,7 | 28,0 | 28,2 | 28,6 |
| | 8-SM | 20,5 | 21,0 | 21,4 | 21,7 | 22,0 | 22,4 | 23,4 | |
| | 8-OD | 25,3 | 26,1 | 26,0 | 26,5 | 27,6 | 27,4 | 28,9 | 28,7 |
| Paclitaxel 15mg/kg q7d4 | 6-SM | 25,6 | 27,1 | 30,7 | 0,0 | 0,0 | 0,0 | 0,0 | 0,0 |
| | 6-OD | 25,8 | 25,7 | 27,8 | 27,7 | 28,0 | 0,0 | 0,0 | 0,0 |
| | 6-OG | 24,5 | 24,3 | 26,2 | 26,5 | 0,0 | 0,0 | 0,0 | 0,0 |
| | 6-2O | 24,4 | 24,6 | 27,7 | 27,2 | 0,0 | 0,0 | 0,0 | 0,0 |
| | 6-2OD | 22,4 | 24,0 | 26,5 | 26,7 | 0,0 | 0,0 | 0,0 | 0,0 |
| | 6-2OG | 25,9 | 26,4 | 29,0 | 28,7 | 0,0 | 0,0 | 0,0 | 0,0 |
| | 10-SM | 24,8 | 25,0 | 26,8 | 27,1 | 26,8 | 0,0 | 0,0 | 0,0 |
| | 10-OD | 23,9 | 23,6 | 23,9 | 23,7 | 25,2 | 26,3 | 0,0 | 0,0 |

(suite)

| Poids corporel individuel | | | | | | | | | |
|---|---|---|---|---|---|---|---|---|---|
| Group | Nombre de souris | D20 | D27 | D35 | D41 | D48 | D55 | D62 | D69 |
| 3C23K 10mg/kg q7d4 + Paclitaxel 15mg/kg q7d4 | 7-SM | 22,0 | 22,4 | 22,3 | 22,3 | 25,5 | 22,2 | 0,0 | 0,0 |
| | 7-OD | 26,4 | 26,8 | 26,8 | 25,7 | 26,7 | 28,3 | 29,4 | 0,0 |
| | 7-OG | 24,1 | 25,1 | 25,9 | 25,6 | 26,3 | 26,2 | 0,0 | 0,0 |
| | 7-2O | 22,0 | 22,2 | 23,7 | 23,4 | 25,1 | 0,0 | 0,0 | 0,0 |
| | 7-2OD | 22,4 | 22,6 | 24,3 | 26,0 | 24,4 | 25,8 | 26,6 | 0,0 |
| | 7-2OG | 22,5 | 23,6 | 25,0 | 25,0 | 24,9 | 26,7 | 27,6 | 0,0 |
| | 10-OG | 22,8 | 23,7 | 24,7 | 23,6 | 24,7 | 24,5 | 24,6 | 25,1 |
| | 10-2O | 22,7 | 23,7 | 24,4 | 23,8 | 24,7 | 25,0 | 25,4 | 26,8 |

Tableau 9 : Résumé des données brutes de volume tumoral individuel

| Groupe | Day | 13 | 20 | 24 | 27 | 31 | 35 | 41 | 45 | 48 | 51 | 55 | 58 | 62 | 66 | 69 | 73 | 76 | 80 | 83 |
|---|---|---|---|---|---|---|---|---|---|---|---|---|---|---|---|---|---|---|---|---|
| **Témoin** | 1-SM | 150 | 403 | 495 | 910 | 840 | 1566 | 1966 | 2320 | | | | | | | | | | | |
| | 1-OD | 98 | 423 | 660 | 1190 | 1568 | 2898 | | | | | | | | | | | | | |
| | 1-OG | 96 | 189 | 399 | 561 | 1344 | 1734 | 2200 | | | | | | | | | | | | |
| | 1-2O | 90 | 210 | 297 | 423 | 520 | 770 | 1584 | 2058 | | | | | | | | | | | |
| | 1-2OG | 74 | 187 | 358 | 576 | 1056 | 1276 | 2474 | | | | | | | | | | | | |
| | 1- | 65 | 162 | 315 | 469 | 725 | 1089 | 1396 | 2128 | | | | | | | | | | | |
| | 10-2OD | 106 | 72 | 41 | 50 | 24 | 117 | 197 | 510 | 819 | 1008 | 2268 | | | | | | | | |
| | 10-2OG | 66 | 105 | 81 | 126 | 143 | 366 | 631 | 725 | 1170 | 1521 | 2145 | | | | | | | | |
| **3C23K 10mg/kg q7d4** | 2-SM | 144 | 105 | 158 | 252 | 184 | 366 | 560 | 918 | 912 | 1485 | 2080 | | | | | | | | |
| | 2-OD | 98 | 135 | 180 | 289 | 404 | 592 | 844 | 1256 | 1672 | 2035 | | | | | | | | | |
| | 2-OG | 98 | 88 | 0 | 0 | 0 | 0 | 0 | 0 | 0 | 0 | 0 | 0 | 0 | 0 | 0 | 0 | 0 | 0 | 0 |
| | 2-2O | 90 | 140 | 102 | 192 | 204 | 488 | 540 | 624 | 551 | 587 | 990 | 1482 | 1080 | 1695 | 1788 | 1820 | 2296 | | |
| | 2-2OD | 76 | 150 | 114 | 168 | 198 | 528 | 634 | 731 | 833 | 900 | 1084 | 1350 | 1440 | 1512 | 1496 | 1559 | 1238 | 1520 | 1615 |
| | 2-2OG | 73 | 60 | 68 | 72 | 68 | 122 | 293 | 392 | 381 | 462 | 600 | 842 | 640 | 666 | 950 | 1018 | 1140 | 1254 | 1463 |
| | 8-SM | 88 | 105 | 84 | 90 | 140 | 268 | 394 | 529 | 689 | 837 | 1309 | 1665 | 2057 | | | | | | |
| | 8-OD | 72 | 108 | 72 | 90 | 28 | 119 | 169 | 166 | 276 | 351 | 600 | 792 | 1215 | 1740 | 2000 | | | | |
| **Paclitaxel 15mg/kg q7d4** | 6-SM | 126 | 424 | 756 | 896 | 1530 | 2706 | | | | | | | | | | | | | |
| | 6-OD | 117 | 243 | 257 | 325 | 420 | 699 | 1064 | 1632 | 1536 | 2166 | | | | | | | | | |
| | 6-OG | 98 | 240 | 356 | 386 | 673 | 1200 | 2380 | | | | | | | | | | | | |
| | 6-2O | 83 | 280 | 284 | 255 | 410 | 1008 | 1309 | 2040 | | | | | | | | | | | |
| | 6-2OD | 83 | 170 | 380 | 560 | 720 | 1653 | 1389 | 2112 | | | | | | | | | | | |
| | 6-2OG | 66 | 126 | 180 | 347 | 410 | 965 | 2269 | | | | | | | | | | | | |
| | 10-SM | 144 | 217 | 180 | 270 | 358 | 823 | 884 | 1197 | 1323 | 2112 | | | | | | | | | |
| | 10-OD | 58 | 105 | 110 | 198 | 192 | 351 | 437 | 798 | 1232 | 1348 | 2132 | | | | | | | | |
| **3C23K 10mg/kg q7d4 + Paclitaxel 15mg/kg q7d4** | 7-SM | 131 | 126 | 85 | 0 | 0 | 0 | 0 | 0 | 0 | 0 | 0 | 0 | 0 | 0 | 0 | 0 | 0 | 0 | 0 |
| | 7-OD | 116 | 105 | 112 | 223 | 160 | 324 | 315 | 390 | 655 | 938 | 1755 | 1710 | 2340 | | | | | | |
| | 7-OG | 95 | 28 | 0 | 0 | 0 | 0 | 0 | 0 | 0 | 0 | 0 | 0 | 0 | 0 | 0 | 0 | 0 | 0 | 0 |
| | 7-2O | 88 | 70 | 142 | 150 | 264 | 768 | 875 | 1033 | 1400 | 2112 | | | | | | | | | |
| | 7-2OD | 74 | 41 | 112 | 105 | 163 | 357 | 368 | 336 | 399 | 532 | 816 | 1140 | 1170 | 2016 | | | | | |
| | 7-2OG | 66 | 41 | 63 | 85 | 63 | 203 | 347 | 281 | 429 | 662 | 924 | 1109 | 1620 | 2081 | | | | | |
| | 10-OG | 110 | 96 | 104 | 96 | 0 | 0 | 0 | 0 | 0 | 0 | 24 | 53 | 50 | 60 | 96 | 189 | 192 | 347 | 413 |
| | 10-2O | 61 | 72 | 72 | 27 | 24 | 66 | 63 | 68 | 105 | 200 | 324 | 551 | 714 | 1040 | 1245 | 1604 | 1725 | 2112 | |

Tableau 10 : Résumé des données brutes de changement en moyenne de poids corporel

| Groups | Jours | 20 | 27 | 35 | 41 | 48 | 55 |
|---|---|---|---|---|---|---|---|
| témoin | Moyenne | 22,83 | 24,24 | 25,88 | 26,07 | | |
| | SD | 0,72 | 0,94 | 1,34 | 1,57 | | |
| 3C23K 10mg/kg q7d4 | Moyenne | 24,15 | 24,78 | 25,63 | 26,05 | 26,06 | 26,04 |
| | SD | 2,22 | 2,21 | 2,36 | 2,60 | 2,32 | 2,01 |
| Paclitaxel 15mg/kg q7d4 | Moyenne | 24,66 | 25,09 | 27,33 | 26,80 | | |
| | SD | 1,17 | 1,22 | 2,02 | 1,55 | | |
| 3C23K 10mg/kg q7d4 + Paclitaxel 15mg/kg q7d4 | Moyenne | 23,11 | 23,76 | 24,64 | 24,43 | 25,29 | 25,53 |
| | SD | 1,48 | 1,54 | 1,36 | 1,33 | 0,82 | 1,91 |

Tableau 11 : Résumé des données brutes des moyennes des volumes tumoraux

| Groups | Jours | 13 | 20 | 24 | 27 | 31 | 35 | 41 | 45 | 48 | 51 | 55 |
|---|---|---|---|---|---|---|---|---|---|---|---|---|
| Témoin | Moyenne | 93 | 219 | 331 | 538 | 777 | 1227 | 1492 | | | | |
| | SD | 10 | 48 | 77 | 142 | 205 | 331 | 339 | | | | |
| 3C23K 10mg/kg q7dx4 | Moyenne | 92 | 111 | 97 | 144 | 153 | 310 | 429 | 577 | 664 | 832 | |
| | SD | 16 | 23 | 41 | 81 | 94 | 183 | 215 | 305 | 362 | 482 | |
| paclitaxel 15mg/kg q7dx4 | Moyenne | 97 | 226 | 313 | 405 | 589 | 1176 | 1390 | | | | |
| | SD | 11 | 38 | 76 | 86 | 157 | 274 | 290 | | | | |
| 3C23K 10mg/kg + paclitaxel 15mg/kg q7dx4 | Moyenne | 92 | 72 | 86 | 86 | 84 | 215 | 246 | 263 | 373 | 555 | 549 |
| | SD | 9 | 13 | 16 | 29 | 38 | 101 | 114 | 132 | 182 | 272 | 269 |

Tableau 12 : Résumé des données brutes des volumes tumoraux médians et des rapports T/C

| Days | 13 | 20 | 24 | 27 | 31 | 35 | 41 | 45 | 48 | 51 | 55 |
|---|---|---|---|---|---|---|---|---|---|---|---|
| **Volume tumoral médian** | | | | | | | | | | | |
| témoin | 93 | 188 | 336 | 515 | 782 | 1183 | 1584 | | | | |
| 3C23K 10mg/kg q7dx4 | 89 | 107 | 93 | 129 | 162 | 317 | 467 | 577 | 620 | 712 | |
| paclitaxel 15mg/kg q7dx4 | 90 | 228 | 270 | 336 | 415 | 986 | 1309 | | | | |
| 3C23K 10mg/kg + paclitaxel 15mg/kg q7dx4 | 91 | 71 | 94 | 90 | 44 | 134 | 189 | 174 | 252 | 366 | 324 |
| **T/C ratio** | | | | | | | | | | | |
| témoin - 3C23K 10mg/kg q7dx4 | 95 | 57 | 28 | 25 | 21 | 27 | 29 | | | | |
| témoin - paclitaxel 15mg/kg q7dx4 | 97 | 121 | 80 | 65 | 53 | 83 | 83 | | | | |
| témoin - 3C23K 10mg/kg q7dx4 + paclitaxel 15mg/kg q7dx4 | 98 | 38 | 28 | 18 | 6 | 11 | 12 | | | | |
| 3C23K 10mg/kg q7dx4 - paclitaxel 15mg/kg q7dx4 | 98 | 47 | 35 | 38 | 39 | 32 | 36 | | | | |

(suite)

| T/C ratio | | | | | | | | | | | |
|---|---|---|---|---|---|---|---|---|---|---|---|
| 3C23K 10mg/kg q7dx4 - 3C23K 10mg/kg q7dx4 + paclitaxel 15mg/kg q7dx4 | 103 | 67 | 101 | 70 | 27 | 42 | 40 | 30 | 41 | 51 | |
| paclitaxel 15mg/kg q7dx4 - 3C23K 10mg/kg + paclitaxel 15mg/kg q7dx4 | 101 | 31 | 35 | 27 | 11 | 14 | 14 | | | | |

Tableau 13 : Analyse statistique

| Jour | Contraste entre groupes | Anova | | | Kruskall wallis | | |
|---|---|---|---|---|---|---|---|
| | | F-ratio | P-value | Sig. | Test | P-value | Sig. |
| D13 | témoin - 3C23K 10 mg/kg q7d4 | 0,00 | 0,95 | | 0,00 | 0,96 | |
| | témoin - paclitaxel 15 mg/kg q7d4 | 0,07 | 0,80 | | 0,04 | 0,83 | |
| | émoin - 3C23K 10 mg/kg q7d4 + paclitaxel 15 mg/kg q7d4 | 0,00 | 0,97 | | 0,00 | 1,00 | |
| | 3C23K 10 mg/kg q7d4 - paclitaxel 15 mg/kg q7d4 | 0,11 | 0,74 | | 0,03 | 0,87 | |
| | 3C23K 10 mg/kg q7d4 - 3C23K 10 mg/kg q7d4 + paclitaxel 15 mg/kg q7d4 | 0,00 | 0,98 | | 0,00 | 0,96 | |
| | paclitaxel 15 mg/kg q7d4 - 3C23K 10 mg/kg q7d4 + paclitaxel 15 mg/kg q7d4 | 0,09 | 0,76 | 0,07 | 0,07 | 0,79 | |
| D20 | témoin- 3C23K 10 mg/kg q7d4 | 5,33 | 0,04 | * | 4,44 | 0,04 | * |
| | témoin - paclitaxel 15 mg/kg q7d4 | 0,01 | 0,91 | | 0,47 | 0,49 | |
| | témoin - 3C23K 10 mg/kg q7d4 + paclitaxel 15 mg/kg q7d4 | 9,71 | 0,01 | * | 8,08 | 0,00 | * |
| | 3C23K 10 mg/kg q7d4 - paclitaxel 15 mg/kg q7d4 | 9,54 | 0,01 | * | 6,39 | 0,01 | * |
| | 3C23K 10 mg/kg q7d4 - 3C23K 10 mg/kg q7d4 + paclitaxel 15 mg/kg q7d4 | 5,84 | 0,03 | * | 4,44 | 0,04 | * |
| | paclitaxel 15 mg/kg q7d4 - 3C23K 10 mg/kg q7d4 + paclitaxel 15 mg/kg q7d4 | 16,67 | 0,00 | * | 9,97 | 0,00 | * |
| D24 | témoin - 3C23K 10 mg/kg q7d4 | 9,83 | 0,01 | * | 4,41 | 0,04 | * |
| | témoin - paclitaxel 15 mg/kg q7d4 | 0,03 | 0,86 | | 0,28 | 0,60 | |
| | témoin - 3C23K 10 mg/kg q7d4 + paclitaxel 15 mg/kg q7d4 | 11,11 | 0,00 | * | 4,42 | 0,04 | * |
| | 3C23K 10 mg/kg q7d4 - paclitaxel 15 mg/kg q7d4 | 8,53 | 0,01 | * | 8,70 | 0,00 | * |
| | 3C23K 10 mg/kg q7d4 - 3C23K 10 mg/kg q7d4 + paclitaxel 15 mg/kg q7d4 | 0,19 | 0,67 | | 0,10 | 0,75 | |
| | paclitaxel 15 mg/kg q7d4 - 3C23K 10 mg/kg q7d4 + paclitaxel 15 mg/kg q7d4 | 9,71 | 0,01 | * | 9,30 | 0,00 | * |

(suite)

| Jour | Contraste entre groupes | Anova | | | Kruskall wallis | | |
|---|---|---|---|---|---|---|---|
| | | F-ratio | P-value | Sig. | Test | P-value | Sig. |
| D27 | témoin - 3C23K 10 mg/kg q7d4 | 8,21 | 0,01 | * | 4,87 | 0,03 | * |
| | témoin - paclitaxel 15 mg/kg q7d4 | 0,74 | 0,40 | | 1,10 | 0,29 | |
| | témoin - 3C23K 10 mg/kg q7d4 + paclitaxel 15 mg/kg q7d4 | 11,09 | 0,00 | * | 6,90 | 0,01 | * |
| | 3C23K 10 mg/kg q7d4 - paclitaxel 15 mg/kg q7d4 | 8,92 | 0,01 | * | 8,66 | 0,00 | * |
| | 3C23K 10 mg/kg q7d4 - 3C23K 10 mg/kg q7d4 + paclitaxel 15 mg/kg q7d4 | 1,75 | 0,21 | 1,11 | 1,11 | 0,29 | |
| | paclitaxel 15 mg/kg q7d4 - 3C23K 10 mg/kg q7d4 + paclitaxel 15 mg/kg q7d4 | 14,22 | 0,00 | * | 10,61 | 0,00 | * |
| D31 | témoin - 3C23K 10 mg/kg q7d4 | 10,00 | 0,01 | * | 4,86 | 0,03 | * |
| | témoin - paclitaxel 15 mg/kg q7d4 | 0,61 | 0,45 | | 0,89 | 0,34 | |
| | témoin - 3C23K 10 mg/kg q7d4 + paclitaxel 15 mg/kg q7d4 | 12,60 | 0,00 | * | 6,67 | 0,01 | * |
| | 3C23K 10 mg/kg q7d4 - paclitaxel 15 mg/kg | 8,01 | 0,01 | * | 8,66 | 0,00 | * |
| | q7d4 | | | | | | |
| | 3C23K 10 mg/kg q7d4 - 3C23K 10 mg/kg q7d4 + paclitaxel 15 mg/kg q7d4 | 1,44 | 0,25 | 1,75 | 1,75 | 0,19 | |
| | paclitaxel 15 mg/kg q7d4 - 3C23K 10 mg/kg q7d4 + paclitaxel 15 mg/kg q7d4 | 11,13 | 0,00 | * | 10,68 | 0,00 | * |
| D35 | témoin - 3C23K 10 mg/kg q7d4 | 8,26 | 0,01 | * | 5,11 | 0,02 | * |
| | témoin - paclitaxel 15 mg/kg q7d4 | 0,02 | 0,90 | | 0,10 | 0,75 | |
| | témoin - 3C23K 10 mg/kg q7d4 + paclitaxel 15 mg/kg q7d4 | 9,78 | 0,01 | * | 8,09 | 0,00 | * |
| | 3C23K 10 mg/kg q7d4 - paclitaxel 15 mg/kg q7d4 | 10,47 | 0,01 | * | 8,65 | 0,00 | * |
| | 3C23K 10 mg/kg q7d4 - 3C23K 10 mg/kg q7d4 + paclitaxel 15 mg/kg q7d4 | 0,62 | 0,45 | | 1,23 | 0,27 | |
| | paclitaxel 15 mg/kg q7d4 - 3C23K 10 mg/kg q7d4 + paclitaxel 15 mg/kg q7d4 | 12,39 | 0,00 | * | 9,33 | 0,00 | * |
| D41 | témoin - 3C23K 10 mg/kg q7d4 | 11,82 | 0,00 | * | 5,36 | 0,02 | * |
| | témoin - paclitaxel 15 mg/kg q7d4 | 0,06 | 0,81 | | 0,20 | 0,65 | |
| | témoin - 3C23K 10 mg/kg q7d4 + paclitaxel 15 mg/kg q7d4 | 15,82 | 0,00 | * | 7,14 | 0,01 | * |
| | 3C23K 10 mg/kg q7d4 - paclitaxel 15 mg/kg q7d4 | 12,64 | 0,00 | * | 7,71 | 0,01 | * |
| | 3C23K 10 mg/kg q7d4 - 3C23K 10 mg/kg q7d4 + paclitaxel 15 mg/kg q7d4 | 1,63 | 0,22 | 1,75 | 1,75 | 0,19 | |
| | paclitaxel 15 mg/kg q7d4 - 3C23K 10 mg/kg q7d4 + paclitaxel 15 mg/kg q7d4 | 17,31 | 0,00 | * | 9,83 | 0,00 | * |
| D45 | 3C23K 10 mg/kg q7d4 - 3C23K 10 mg/kg q7d4 + paclitaxel 15 mg/kg q7d4 | 2,75 | 0,12 | 3,05 | 3,05 | 0,08 | |
| D48 | 3C23K 10 mg/kg q7d4 - 3C23K 10 mg/kg q7d4 + paclitaxel 15 mg/kg q7d4 | 1,38 | 0,26 | 1,75 | 1,75 | 0,19 | |

(suite)

| Jour | Contraste entre groupes | Anova | | | Kruskall wallis | | |
|---|---|---|---|---|---|---|---|
| | | F-ratio | P-value | Sig. | Test | P-value | Sig. |
| D51 | 3C23K 10 mg/kg q7d4 - 3C23K 10 mg/kg q7d4 + paclitaxel 15 mg/kg q7d4 | 0,65 | 0,43 | 0,81 | 0,81 | 0,37 | |

**Tableau 14 : Résumé des données brutes de paramètre de survie**

| Nombre souris | Groupes | Jours | Observations |
|---|---|---|---|
| 1 | NaCl | 34 | |
| 2 | NaCl | 41 | |
| 3 | NaCl | 41 | |
| 4 | NaCl | 45 | |
| 5 | NaCl | 45 | |
| 6 | NaCl | 45 | |
| 7 | NaCl | 55 | |
| 8 | NaCl | 55 | |
| 1 | 3C23K 10 mg/kg q7d4 | 51 | |
| 2 | 3C23K 10 mg/kg q7d4 | 55 | |
| 3 | 3C23K 10 mg/kg q7d4 | 62 | |
| 4 | 3C23K 10 mg/kg q7d4 | 69 | |
| 5 | 3C23K 10 mg/kg q7d4 | 76 | |
| 6 | 3C23K 10 mg/kg q7d4 | 83 | Fin d'expérience TV 1615mm3 |
| 7 | 3C23K 10 mg/kg q7d4 | 83 | Fin d'expérience TV 1463mm3 |
| 8 | 3C23K 10 mg/kg q7d4 | 83 | Fin d'expérience pas de tumeur |
| 1 | Paclitaxel 15mg/kg q7d4 | 34 | |
| 2 | Paclitaxel 15mg/kg q7d4 | 41 | |
| 3 | Paclitaxel 15mg/kg q7d4 | 41 | |
| 4 | Paclitaxel 15mg/kg q7d4 | 45 | |
| 5 | Paclitaxel 15mg/kg q7d4 | 45 | |
| 6 | Paclitaxel 15mg/kg q7d4 | 51 | |
| 7 | Paclitaxel 15mg/kg q7d4 | 51 | |
| 8 | Paclitaxel 15mg/kg q7d4 | 55 | |
| 1 | 3C23K 10 mg/kg q7d4 + Paclitaxel 15mg/kg q7d4 | 51 | |
| 2 | 3C23K 10 mg/kg q7d4 + Paclitaxel 15mg/kg q7d4 | 62 | |
| 3 | 3C23K 10 mg/kg q7d4 + Paclitaxel 15mg/kg q7d4 | 66 | |
| 4 | 3C23K 10 mg/kg q7d4 + Paclitaxel 15mg/kg q7d4 | 66 | |
| 5 | 3C23K 10 mg/kg q7d4 + Paclitaxel 15mg/kg q7d4 | 80 | |
| 6 | 3C23K 10 mg/kg q7d4 + Paclitaxel 15mg/kg q7d4 | 83 | Fin d'expérience TV 413mm3 |
| 7 | 3C23K 10 mg/kg q7d4 + Paclitaxel 15mg/kg q7d4 | 83 | Fin d'expérience Pas de tumeur |
| 8 | 3C23K 10 mg/kg q7d4 + Paclitaxel 15mg/kg q7d4 | 83 | Fin d'expérience Pas de tumeur |

**Tableau 15 : paramètres de survie**

| Paramètre de survie | | | |
|---|---|---|---|
| Groupe | Moyenne | Médian | SD |
| Témoin | 45 | 45 | 5 |
| 3C23K 10mg/kg q7dx4 | 70 | 73 | 11 |
| Paclitaxel 15mg/kg q7dx4 | 45 | 45 | 5 |
| 3C23K 10mg/kg + Paclitaxel 15mg/kg q7dx4 | 72 | 72 | 11 |
| Paramètre de survie: test de logrank | | | |
| | chi-squared | p-value | sign. |
| Témoin - 3C23K 10 mg/kg q7d4 | 11,15 | 0,00 | * |
| Témoin - paclitaxel 15 mg/kg q7d4 | 0,04 | 0,84 | |
| Témoin - 3C23K 10 mg/kg q7d4 + paclitaxel 15 mg/kg q7d4 | 12,87 | 0,00 | * |
| 3C23K 10 mg/kg q7d4 - paclitaxel 15 mg/kg q7d4 | 12,36 | 0,00 | * |
| 3C23K 10 mg/kg q7d4 - 3C23K 10 mg/kg q7d4 + paclitaxel 15 mg/kg q7d4 | 0,17 | 0,68 | |
| paclitaxel 15 mg/kg q7d4 - 3C23K 10 mg/kg q7d4 + paclitaxel 15 mg/kg q7d4 | 13,61 | 0,00 | * |

## Revendications

**1.** Composition pharmaceutique pour son utilisation comme médicament dans la prévention ou le traitement du cancer ovarien comprenant, à titre de substance active, en association avec un véhicule pharmaceutiquement acceptable,

- un agent anti-cancéreux, lequel est une anthracycline choisie parmi le groupe constitué par la doxorubicine et la doxorubicine liposomale pégylée ;
- un anticorps liant le récepteur de type II de l'hormone anti-mullérienne humaine (AMHR-II), lequel est un anticorps monoclonal humanisé 12G4 muté comprenant ou constitué :

a) d'une chaîne légère comprenant ou constituée :

- d'une région variable dont la séquence en acides aminés est représentée par SEQ ID NO : 7 ou SEQ ID NO : 8, et
- d'une région constante dont la séquence en acides aminés est représentée par SEQ ID NO : 3; et

b) d'une chaîne lourde comprenant ou constituée :

- d'une région variable dont la séquence en acides aminés est représentée par SEQ ID NO : 9 ou SEQ ID NO : 10, et
- d'une région constante dont la séquence en acides aminés est représentée par SEQ ID NO : 6.

**2.** Composition pharmaceutique pour son utilisation selon la revendication 1, dans laquelle ledit anticorps monoclonal possède notamment une affinité pour AMHR-II **caractérisée par** un $K_D$ préférentiellement inférieur à $10^{-7}$ M, notamment inférieur à $10^{-8}$ M, en particulier compris de $10^{-9}$ M à $10^{-11}$ M.

**3.** Composition pharmaceutique pour son utilisation selon la revendication 1 ou 2, dans laquelle l'anticorps est un anticorps recombinant produit par transgénèse animale.

**4.** Composition pharmaceutique pour son utilisation selon l'une des revendications 1 à 3, dans une formulation destinée à une administration par voie intra-veineuse ou intra-péritonéale.

**5.** Composition pharmaceutique pour son utilisation selon l'une des revendications 1 à 4, dans laquelle la quantité thérapeutiquement efficace d'anticorps administrée à un patient est comprise dans une gamme d'environ 0,07 mg à environ 35000 mg, de préférence d'environ 0,7 mg à environ 7000 mg, de préférence d'environ 0,7 mg à environ 1400 mg, de préférence d'environ 0,7 mg à environ 700 mg, et plus préférentiellement d'environ 0,7 mg à environ 70 mg.

**6.** Composition pharmaceutique pour son utilisation selon l'une des revendications 1 à 5, dans laquelle la quantité thérapeutiquement efficace d'agent anticancéreux administrée à un patient est comprise dans une gamme d'environ 10 mg à environ 700 mg, de préférence dans une gamme d'environ 20 mg à environ 350 mg, et de préférence d'environ 110 mg.

**7.** Composition pharmaceutique pour son utilisation selon l'une des revendications 1 à 6, dans laquelle la dose d'anticorps administrée à un patient est d'environ 70 mg et la dose d'agent anticancéreux administrée au patient est d'environ 110 mg.

## Patentansprüche

**1.** Pharmazeutische Zusammensetzung zu ihrer Verwendung als Medikament bei der Prävention oder der Behandlung von Eierstockkrebs, umfassend, als aktive Substanz, in Assoziation mit einem pharmazeutisch annehmbaren Träger,

- ein Anti-Krebsmittel, das ein Anthracyclin ist, ausgewählt aus der Gruppe bestehend aus Doxorubicin und pegyliertem liposomalen Doxorubicin;
- einen Antikörper, der den menschlichen Anti-Müller-Hormonrezeptor Typ II (AMHR-II) bindet, der ein mutierter humanisierter monoklonaler Antikörper 12G4 ist, umfassend oder bestehend aus:

a) einer leichten Kette, umfassend oder bestehend aus:

- einer variablen Region, deren Aminosäuresequenz durch die SEQ ID NR : 7 oder SEQ ID NR : 8 dargestellt wird, und
- einer konstanten Region, deren Aminosäuresequenz durch die SEQ ID NR : 3 dargestellt wird; und

b) einer schweren Kette, umfassend oder bestehend aus:

- einer variablen Region, deren Aminosäuresequenz durch die SEQ ID NR : 9 oder SEQ ID NR : 10 dargestellt wird, und
- einer konstanten Region, deren Aminosäuresequenz durch die SEQ ID NR : 6 dargestellt wird.

**2.** Pharmazeutische Zusammensetzung zu ihrer Verwendung nach Anspruch 1, wobei der monoklonale Antikörper insbesondere eine Affinität für AMHR-II aufweist, **gekennzeichnet durch** eine $K_D$ von vorzugsweise weniger als $10^{-7}$ M, insbesondere weniger als $10^{-8}$ M, vor allem zwischen $10^{-9}$ M bis $10^{-11}$ M.

**3.** Pharmazeutische Zusammensetzung zu ihrer Verwendung nach einem der Ansprüche 1 oder 2, wobei der Antikörper ein rekombinanter Antikörper ist, der durch tierische Transgenese erzeugt wird.

**4.** Pharmazeutische Zusammensetzung zu ihrer Verwendung nach einem der Ansprüche 1 bis 3, in einer Formulierung, die zur Verabreichung auf intravenösem oder intraperitonealem Weg bestimmt ist.

**5.** Pharmazeutische Zusammensetzung zu ihrer Verwendung nach einem der Ansprüche 1 bis 4, wobei die therapeutisch wirksame Menge an Antikörper, die einem Patienten verabreicht wird, in einem Bereich von ungefähr 0,07 mg bis ungefähr 35000 mg, vorzugsweise von ungefähr 0,7 mg bis ungefähr 7000 mg, vorzugsweise von ungefähr 0,7 mg bis ungefähr 1400 mg, vorzugsweise von ungefähr 0,7 mg bis ungefähr 700 mg, und bevorzugter von ungefähr 0,7 mg bis ungefähr 70 mg, liegt.

**6.** Pharmazeutische Zusammensetzung zu ihrer Verwendung nach einem der Ansprüche 1 bis 5, wobei die therapeutisch wirksame Menge an Anti-Krebsmittel, die einem Patienten verabreicht wird, in einem Bereich von ungefähr 10 mg bis ungefähr 700 mg, vorzugsweise in einem Bereich von ungefähr 20 mg bis ungefähr 350 mg, und vorzugsweise ungefähr 110 mg, liegt.

**7.** Pharmazeutische Zusammensetzung zu ihrer Verwendung nach einem der Ansprüche 1 bis 6, wobei die Dosis an Antikörper, die einem Patienten verabreicht wird, ungefähr 70 mg beträgt, und die Dosis an Anti-Krebsmittel, die einem Patienten verabreicht wird, ungefähr 110 mg beträgt.

## Claims

**1.** Pharmaceutical composition for its use as a drug in the prevention or treatment of ovarian cancer comprising, as active ingredient, in association with a pharmaceutically acceptable vehicle,

- an anticancer agent, which is an anthracycline selected from the group consisting of doxorubicin and pegylated liposomal doxorubicin;
- an antibody binding the human anti-Müllerian hormone type II receptor (AMHR-II), which is a mutated humanized 12G4 monoclonal antibody comprising or constituted by:

  a) a light chain comprising or constituted by:

    - a variable region the amino acid sequence of which is represented by SEQ ID NO: 7 or SEQ ID NO: 8, and
    - a constant region the amino acid sequence of which is represented by SEQ ID NO: 3; and

  b) a heavy chain comprising or constituted by:

    - a variable region the amino acid sequence of which is represented by SEQ ID NO: 9 or SEQ ID NO: 10, and
    - a constant region the amino acid sequence of which is represented by SEQ ID NO: 6.

**2.** Pharmaceutical composition for its use according to claim 1, in which said monoclonal antibody in particular possesses affinity for AMHR-II **characterized by** a $K_D$ preferably less than $10^{-7}$ M, in particular less than $10^{-8}$ M, in particular in the range from $10^{-9}$ M to $10^{-11}$ M.

**3.** Pharmaceutical composition for its use according to claim 1 or 2, in which the antibody is a recombinant antibody produced by animal transgenesis.

**4.** Pharmaceutical composition for its use according to one of claims 1 to 3, in a formulation intended for administration by the intravenous or intraperitoneal route.

**5.** Pharmaceutical composition for its use according to one of claims 1 to 4, in which the therapeutically effective quantity of antibody administered to a patient is in a range from about 0.07 mg to about 35000 mg, preferably from about 0.7 mg to about 7000 mg, preferably from about 0.7 mg to about 1400 mg, preferably from about 0.7 mg to about 700 mg, and more preferably from about 0.7 mg to about 70 mg.

**6.** Pharmaceutical composition for its use according to one of claims 1 to 5, in which the therapeutically effective quantity of anticancer agent administered to a patient is in a range from about 10 mg to about 700 mg, preferably in a range from about 20 mg to about 350 mg, and preferably is about 110 mg.

**7.** Pharmaceutical composition for its use according to one of claims 1 to 6, in which the dose of antibody administered to a patient is about 70 mg and the dose of anticancer agent administered to the patient is about 110 mg.

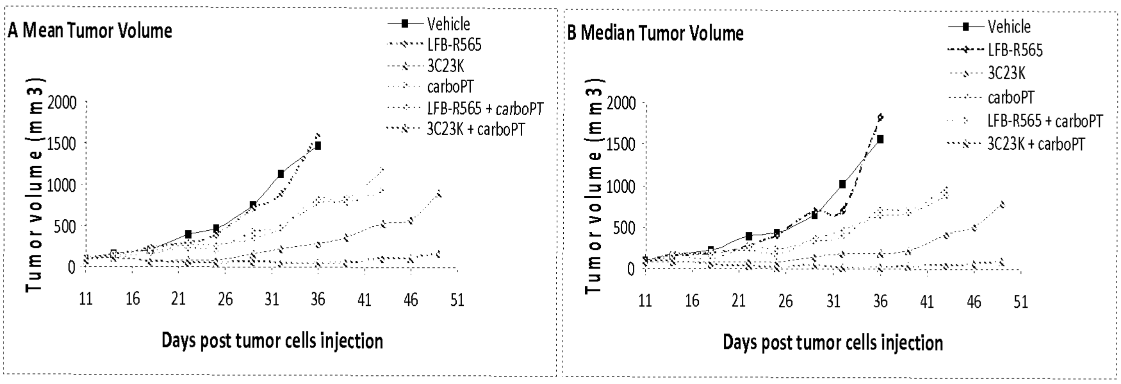
A Mean Tumor Volume
Vehicle
LFB-R565
3C23K
carboPT
LFB-R565 + carboPT
3C23K + carboPT
Tumor volume (mm3)
2000
1500
1000
500
0
11
16
21
26
31
36
41
46
51
Days post tumor cells injection
B Median Tumor Volume
Vehicle
LFB-R565
3C23K
carboPT
LFB-R565 + carboPT
3C23K + carboPT
Tumor volume (mm3)
2000
1500
1000
500
0
11
16
21
26
31
36
41
46
51
Days post tumor cells injection

Figure 1

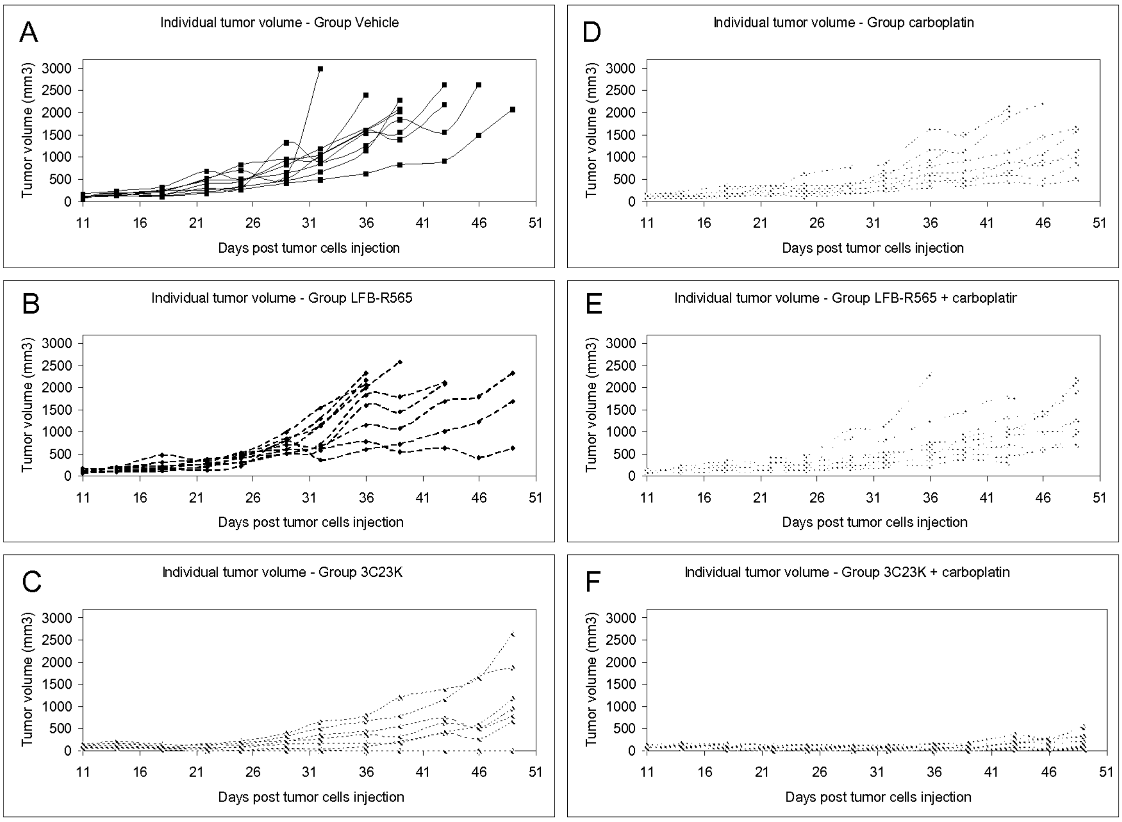
A
Individual tumor volume - Group Vehicle
B
Individual tumor volume - Group LFB-R565
C
Individual tumor volume - Group 3C23K
D
Individual tumor volume - Group carboplatin
E
Individual tumor volume - Group LFB-R565 + carboplatin
F
Individual tumor volume - Group 3C23K + carboplatin
Tumor volume (mm3)
Days post tumor cells injection

Figure 2

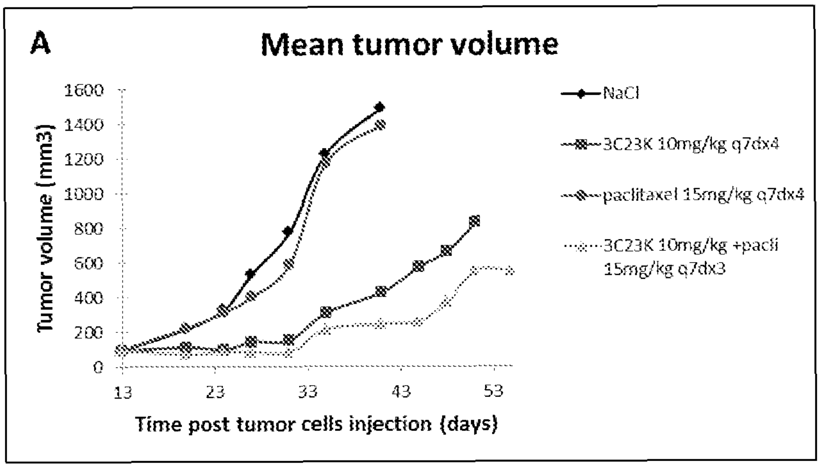
A
Mean tumor volume
Tumor volume (mm3)
Time post tumor cells injection (days)
NaCl
3C23K 10mg/kg q7dx4
paclitaxel 15mg/kg q7dx4
3C23K 10mg/kg +pacli 15mg/kg q7dx3

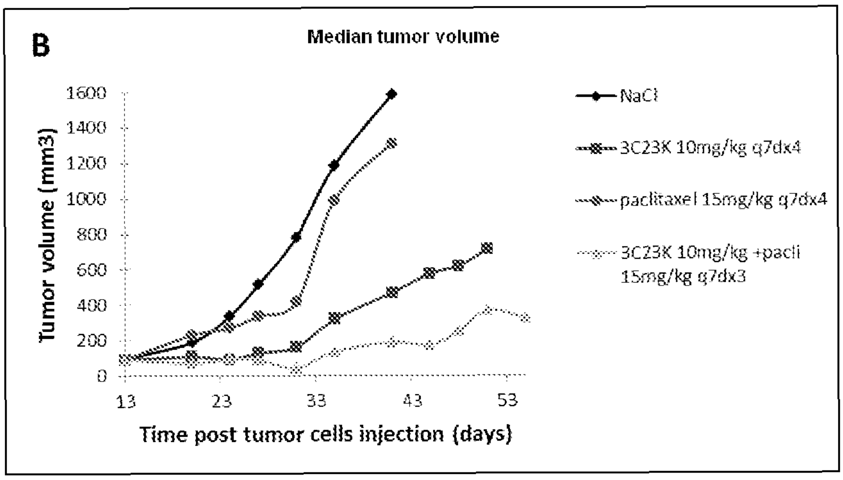
B
Median tumor volume
Tumor volume (mm3)
Time post tumor cells injection (days)
NaCl
3C23K 10mg/kg q7dx4
paclitaxel 15mg/kg q7dx4
3C23K 10mg/kg +pacli 15mg/kg q7dx3

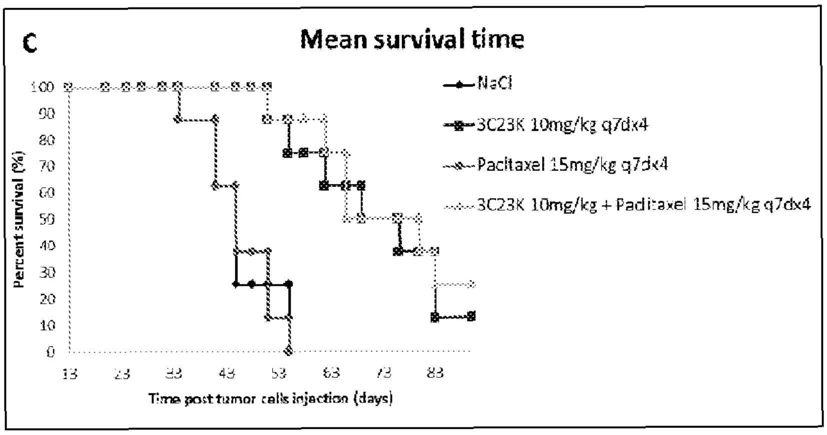
C
Mean survival time
Percent survival (%)
Time post tumor cells injection (days)
NaCl
3C23K 10mg/kg q7dx4
Paclitaxel 15mg/kg q7dx4
3C23K 10mg/kg + Paclitaxel 15mg/kg q7dx4

Figure 3

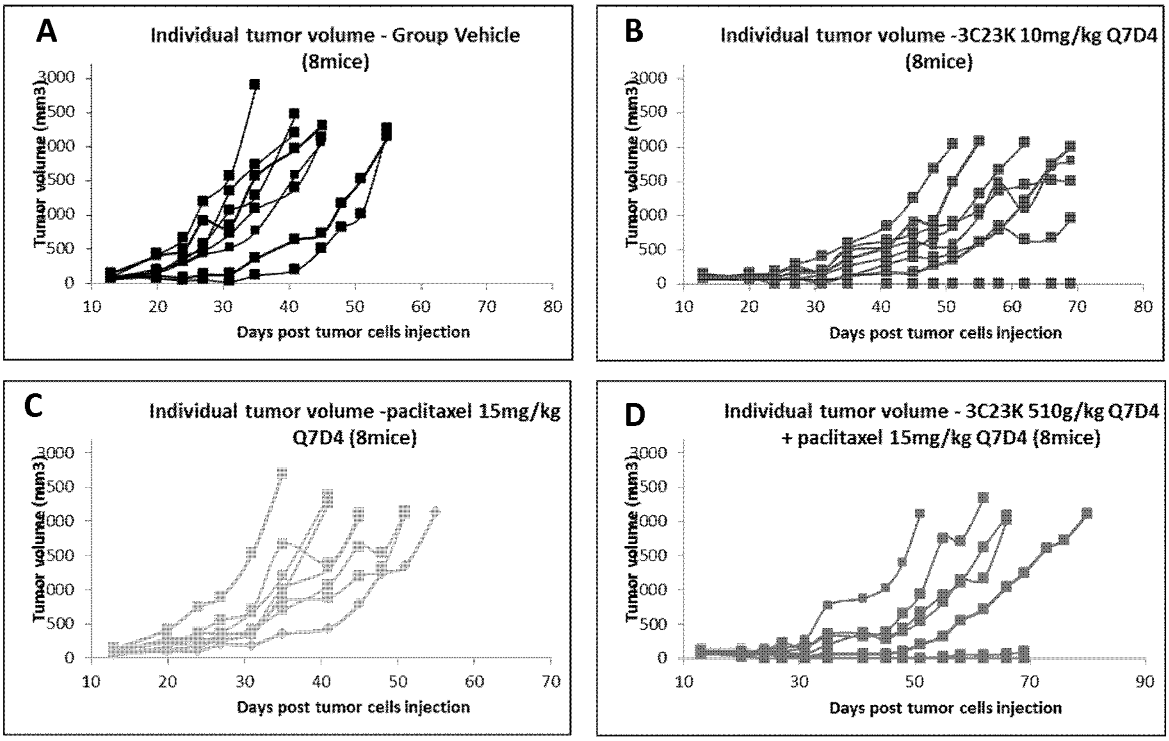
A
Individual tumor volume - Group Vehicle (8mice)
Tumor volume (mm3)
Days post tumor cells injection
B
Individual tumor volume -3C23K 10mg/kg Q7D4 (8mice)
Tumor volume (mm3)
Days post tumor cells injection
C
Individual tumor volume -paclitaxel 15mg/kg Q7D4 (8mice)
Tumor volume (mm3)
Days post tumor cells injection
D
Individual tumor volume - 3C23K 510g/kg Q7D4 + paclitaxel 15mg/kg Q7D4 (8mice)
Tumor volume (mm3)
Days post tumor cells injection

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2008053330 A
- WO 2011141653 A
- US 4816567 A
- US 6331415 B
- US 6808901 B
- EP 125023 A
- FR 2641468
- US 5204244 A
- US 5202238 A
- US 5225539 A
- US 5585089 A
- EP 0682040 A
- WO 2006082406 A
- US 5639641 A
- EP 0571613 A
- US 5766886 A
- US 5770196 A
- US 5821123 A
- US 5869619 A


### Littérature non-brevet citée dans la description


- **LA MARCA A. ; VOLPE A.** The Anti-Mullerian hormone and ovarian cancer. *Human Reproduction Update,* 2007, vol. 13 (3), 265-273
- **FAUCI, BRAUNWALD et al.** Principles of internai medicine. National Cancer Institute
- **EGFR, OZOLS R. F. et al.** Focus on epithelial ovarian cancer. *Cancer Cell,* Janvier 2004, vol. 5 (1), 19-24
- **MORRISON et al.** *Proc. Natl. Acad. Sci. U.S.A.,* 1984, vol. 81, 6851-55
- **BOULIANNE, G. L. et al.** *Nature,* 1984, vol. 312, 643-646
- **SUN, L.K. et al.** *Proc. Natl. Acad. Sci. USA,* 1984, 214-218
- **BOBRZECKA, K. et al.** *Immunology Letters,* vol. 2, 151-155
- **G.T. STEVENSON et al.** *Med. Oncol. & Tumor,* 1985
- *Pharmacother,* 1984, vol. 1 (4), 275-278
- **JONES et al.** *Nature,* 1986, 321-522, 525
- **SINGER et al.** *J. Immun.,* 1993, vol. 150, 2844-2857
- **RIECHMAN et al.** *Nature,* 1988, vol. 332 (323), 326
- **TAMURA et al.** *J Immunol.,* 2000, vol. 164, 1432-41
- **PADLAN et al.** *FASEB J.,* 1995, vol. 9, 133-139
- **PASCALIS et al.** *The Journal of Immunology,* 2002, vol. 169, 3076-3084
- **KASHMIRI SYED V.S et al.** *Methods,* Mai 2005, vol. 36, 25-34
- **ROGUSKA et al.** *Proc Natl Acad Sci USA,* 1994
- The pharmacology of monoclonal Antibodies. **MARK G. E. et al.** Handbook of Experimental Pharmacology. Springer-Verlag, 1994, vol. 113, 105-134

SEQUENCE LISTING

<110> LFB Biotechnologies

<120> Nouvelles compositions pharmaceutiques comprenant un anticorps liant le recepteur humain de l'hormone anti-mullérienne de type II

<130> WOB 11 DA LFB AMAC

<160> 14

<170> PatentIn version 3.5

<210> 1
<211> 106
<212> PRT
<213> Artificial Sequence

<220>
<223> VL sans leader 12G4 humanisé

<220>
<221> PEPTIDE
<222> (1)..(106)

<400> 1

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Ile Tyr
        35                  40                  45

Ser Thr Ser Ser Leu Glu Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys
            100                 105
```

<210> 2
<211> 128
<212> PRT
<213> Artificial Sequence

<220>
<223> VL avec leader 12G4 humanisé

<220>
<221> PEPTIDE
<222> (1)..(128)

<400> 2

Met Asp Met Arg Val Pro Ala Gln Leu Leu Gly Leu Leu Leu Leu Trp
1               5                   10                  15

Leu Pro Gly Ala Lys Cys Asp Ile Gln Met Thr Gln Ser Pro Ser Thr
            20                  25                  30

Leu Ser Ala Ser Val Gly Asp Arg Val Thr Ile Thr Cys Arg Ala Ser
        35                  40                  45

Ser Ser Val Arg Tyr Ile Ala Trp Tyr Gln Gln Lys Pro Gly Lys Ala
    50                  55                  60

Pro Lys Leu Leu Ile Tyr Ser Thr Ser Ser Leu Glu Ser Gly Val Pro
65                  70                  75                  80

Ser Arg Phe Ser Gly Ser Gly Ser Gly Thr Glu Phe Thr Leu Thr Ile
                85                  90                  95

Ser Ser Leu Gln Pro Asp Asp Phe Ala Thr Tyr Tyr Cys Leu Gln Trp
            100                 105                 110

Ser Ser Tyr Pro Trp Thr Phe Gly Gly Gly Thr Lys Val Glu Ile Lys
        115                 120                 125

<210> 3
<211> 107
<212> PRT
<213> Artificial Sequence

<220>
<223> CL 12G4 humanisé

<400> 3

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 4
<211> 115
<212> PRT
<213> Artificial Sequence

<220>
<223> VH sans leader 12G4 humanisé

<220>
<221> PEPTIDE
<222> (1)..(115)

<400> 4

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

His Ile His Trp Val Arg Gln Ala Pro Gly Gln Arg Leu Glu Trp Met
35 40 45

Gly Trp Ile Tyr Pro Gly Asp Asp Ser Thr Lys Tyr Ser Gln Lys Phe
50 55 60

Gln Gly Arg Val Thr Ile Thr Arg Asp Thr Ser Ala Ser Thr Ala Tyr
65 70 75 80

Met Glu Leu Ser Ser Leu Arg Ser Glu Asp Thr Ala Val Tyr Tyr Cys
85 90 95

Thr Arg Gly Asp Arg Phe Ala Tyr Trp Gly Gln Gly Thr Leu Val Thr
100 105 110

Val Ser Ser
115

<210> 5
<211> 134
<212> PRT
<213> Artificial Sequence

<220>
<223> VH avec leader 12G4 humanisé

<220>
<221> PEPTIDE
<222> (1)..(134)

<400> 5

```
Met Asp Trp Thr Trp Arg Ile Leu Phe Leu Val Ala Ala Ala Thr Gly
1               5                   10                  15

Ala His Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Leu
    50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
                85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
        115                 120                 125

Leu Val Thr Val Ser Ser
    130
```

<210> 6
<211> 330
<212> PRT
<213> Artificial Sequence

<220>
<223> CH 12G4 humanisé

<220>
<221> PEPTIDE
<222> (1)..(330)

<400> 6

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240
```

```
Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 7
<211> 106
<212> PRT
<213> Artificial Sequence

<220>
<223> VL sans leader 3C_23K

<220>
<221> PEPTIDE
<222> (1)..(106)

<400> 7

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Thr Tyr
        35                  40                  45

Pro Thr Ser Ser Leu Lys Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95
```

Phe Gly Gly Gly Thr Lys Val Glu Ile Lys
100 105

<210> 8
<211> 128
<212> PRT
<213> Artificial Sequence

<220>
<223> VL avec leader 3C_23K

<220>
<221> PEPTIDE
<222> (1)..(128)

<400> 8

Met Asp Met Arg Val Pro Ala Gln Leu Leu Gly Leu Leu Leu Leu Trp
1 5 10 15

Leu Pro Gly Ala Lys Cys Asp Ile Gln Met Thr Gln Ser Pro Ser Thr
20 25 30

Leu Ser Ala Ser Val Gly Asp Arg Val Thr Ile Thr Cys Arg Ala Ser
35 40 45

Ser Ser Val Arg Tyr Ile Ala Trp Tyr Gln Gln Lys Pro Gly Lys Ala
50 55 60

Pro Lys Leu Leu Thr Tyr Pro Thr Ser Ser Leu Lys Ser Gly Val Pro
65 70 75 80

Ser Arg Phe Ser Gly Ser Gly Ser Gly Thr Glu Phe Thr Leu Thr Ile
85 90 95

Ser Ser Leu Gln Pro Asp Asp Phe Ala Thr Tyr Tyr Cys Leu Gln Trp
100 105 110

Ser Ser Tyr Pro Trp Thr Phe Gly Gly Gly Thr Lys Val Glu Ile Lys
115 120 125

<210> 9
<211> 115
<212> PRT
<213> Artificial Sequence

<220>
<223> VH sans leader 3C_23K

<220>
<221> PEPTIDE
<222> (1)..(115)

<400> 9

```
Gln Val Arg Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

His Ile His Trp Val Arg Gln Ala Pro Gly Gln Arg Leu Glu Trp Met
        35                  40                  45

Gly Trp Ile Tyr Pro Gly Asp Asp Ser Thr Lys Tyr Ser Gln Lys Phe
    50                  55                  60

Gln Gly Arg Val Thr Ile Thr Arg Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Asp Arg Phe Ala Tyr Trp Gly Gln Gly Thr Leu Val Thr
            100                 105                 110

Val Ser Ser
        115
```

<210> 10
<211> 134
<212> PRT
<213> Artificial Sequence

<220>
<223> VH avec leader 3C_23K

<220>
<221> PEPTIDE
<222> (1)..(134)

<400> 10

```
Met Asp Trp Thr Trp Arg Ile Leu Phe Leu Val Ala Ala Ala Thr Gly
1               5                   10                  15

Ala His Ser Gln Val Arg Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Leu

    50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
                85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
        115                 120                 125

Leu Val Thr Val Ser Ser
    130
```

<210> 11
<211> 213
<212> PRT
<213> Artificial Sequence

<220>
<223> Chaîne légère sans leader 3C_23K

<220>
<221> PEPTIDE
<222> (1)..(213)

<400> 11

```
Asp Ile Gln Met Thr Gln Ser Pro Ser Thr Leu Ser Ala Ser Val Gly
1               5                   10                  15

Asp Arg Val Thr Ile Thr Cys Arg Ala Ser Ser Ser Val Arg Tyr Ile
            20                  25                  30

Ala Trp Tyr Gln Gln Lys Pro Gly Lys Ala Pro Lys Leu Leu Thr Tyr
        35                  40                  45

Pro Thr Ser Ser Leu Lys Ser Gly Val Pro Ser Arg Phe Ser Gly Ser
    50                  55                  60

Gly Ser Gly Thr Glu Phe Thr Leu Thr Ile Ser Ser Leu Gln Pro Asp
65                  70                  75                  80

Asp Phe Ala Thr Tyr Tyr Cys Leu Gln Trp Ser Ser Tyr Pro Trp Thr
                85                  90                  95

Phe Gly Gly Gly Thr Lys Val Glu Ile Lys Arg Thr Val Ala Ala Pro

            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210
```

<210> 12
<211> 235
<212> PRT
<213> Artificial Sequence

<220>
<223> Chaîne légère avec leader 3C_23K

<220>
<221> PEPTIDE
<222> (1)..(235)

<400> 12

```
Met Asp Met Arg Val Pro Ala Gln Leu Leu Gly Leu Leu Leu Leu Trp
1               5                   10                  15

Leu Pro Gly Ala Lys Cys Asp Ile Gln Met Thr Gln Ser Pro Ser Thr
            20                  25                  30

Leu Ser Ala Ser Val Gly Asp Arg Val Thr Ile Thr Cys Arg Ala Ser
        35                  40                  45

Ser Ser Val Arg Tyr Ile Ala Trp Tyr Gln Gln Lys Pro Gly Lys Ala
    50                  55                  60

Pro Lys Leu Leu Thr Tyr Pro Thr Ser Ser Leu Lys Ser Gly Val Pro
```

65 70 75 80

Ser Arg Phe Ser Gly Ser Gly Ser Gly Thr Glu Phe Thr Leu Thr Ile
85 90 95

Ser Ser Leu Gln Pro Asp Asp Phe Ala Thr Tyr Tyr Cys Leu Gln Trp
100 105 110

Ser Ser Tyr Pro Trp Thr Phe Gly Gly Gly Thr Lys Val Glu Ile Lys
115 120 125

Arg Thr Val Ala Ala Pro Ser Val Phe Ile Phe Pro Pro Ser Asp Glu
130 135 140

Gln Leu Lys Ser Gly Thr Ala Ser Val Val Cys Leu Leu Asn Asn Phe
145 150 155 160

Tyr Pro Arg Glu Ala Lys Val Gln Trp Lys Val Asp Asn Ala Leu Gln
165 170 175

Ser Gly Asn Ser Gln Glu Ser Val Thr Glu Gln Asp Ser Lys Asp Ser
180 185 190

Thr Tyr Ser Leu Ser Ser Thr Leu Thr Leu Ser Lys Ala Asp Tyr Glu
195 200 205

Lys His Lys Val Tyr Ala Cys Glu Val Thr His Gln Gly Leu Ser Ser
210 215 220

Pro Val Thr Lys Ser Phe Asn Arg Gly Glu Cys
225 230 235

<210> 13
<211> 445
<212> PRT
<213> Artificial Sequence

<220>
<223> Chaîne lourde sans leader 3C_23K

<220>
<221> PEPTIDE
<222> (1)..(445)

<400> 13

Gln Val Arg Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr

20 25 30

His Ile His Trp Val Arg Gln Ala Pro Gly Gln Arg Leu Glu Trp Met
35 40 45

Gly Trp Ile Tyr Pro Gly Asp Asp Ser Thr Lys Tyr Ser Gln Lys Phe
50 55 60

Gln Gly Arg Val Thr Ile Thr Arg Asp Thr Ser Ala Ser Thr Ala Tyr
65 70 75 80

Met Glu Leu Ser Ser Leu Arg Ser Glu Asp Thr Ala Val Tyr Tyr Cys
85 90 95

Thr Arg Gly Asp Arg Phe Ala Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

Thr Gln Thr Tyr Ile Cys Asn Val Asn His Lys Pro Ser Asn Thr Lys
195 200 205

Val Asp Lys Lys Val Glu Pro Lys Ser Cys Asp Lys Thr His Thr Cys
210 215 220

Pro Pro Cys Pro Ala Pro Glu Leu Leu Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 14
<211> 464
<212> PRT
<213> Artificial Sequence

<220>
<223> Chaîne lourde avec leader 3C_23K

<220>
<221> PEPTIDE
<222> (1)..(464)

<400> 14

Met Asp Trp Thr Trp Arg Ile Leu Phe Leu Val Ala Ala Ala Thr Gly
1 5 10 15

```
Ala His Ser Gln Val Arg Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr His Ile His Trp Val Arg Gln Ala Pro Gly Gln Arg Leu
    50                  55                  60

Glu Trp Met Gly Trp Ile Tyr Pro Gly Asp Asp Ser Thr Lys Tyr Ser
65                  70                  75                  80

Gln Lys Phe Gln Gly Arg Val Thr Ile Thr Arg Asp Thr Ser Ala Ser
                85                  90                  95

Thr Ala Tyr Met Glu Leu Ser Ser Leu Arg Ser Glu Asp Thr Ala Val
            100                 105                 110

Tyr Tyr Cys Thr Arg Gly Asp Arg Phe Ala Tyr Trp Gly Gln Gly Thr
        115                 120                 125

Leu Val Thr Val Ser Ser Ala Ser Thr Lys Gly Pro Ser Val Phe Pro
    130                 135                 140

Leu Ala Pro Ser Ser Lys Ser Thr Ser Gly Gly Thr Ala Ala Leu Gly
145                 150                 155                 160

Cys Leu Val Lys Asp Tyr Phe Pro Glu Pro Val Thr Val Ser Trp Asn
                165                 170                 175

Ser Gly Ala Leu Thr Ser Gly Val His Thr Phe Pro Ala Val Leu Gln
            180                 185                 190

Ser Ser Gly Leu Tyr Ser Leu Ser Ser Val Val Thr Val Pro Ser Ser
        195                 200                 205

Ser Leu Gly Thr Gln Thr Tyr Ile Cys Asn Val Asn His Lys Pro Ser
    210                 215                 220

Asn Thr Lys Val Asp Lys Lys Val Glu Pro Lys Ser Cys Asp Lys Thr
225                 230                 235                 240

His Thr Cys Pro Pro Cys Pro Ala Pro Glu Leu Leu Gly Gly Pro Ser
                245                 250                 255

Val Phe Leu Phe Pro Pro Lys Pro Lys Asp Thr Leu Met Ile Ser Arg
            260                 265                 270
```

```
Thr Pro Glu Val Thr Cys Val Val Val Asp Val Ser His Glu Asp Pro
        275                 280                 285

Glu Val Lys Phe Asn Trp Tyr Val Asp Gly Val Glu Val His Asn Ala
    290                 295                 300

Lys Thr Lys Pro Arg Glu Glu Gln Tyr Asn Ser Thr Tyr Arg Val Val
305                 310                 315                 320

Ser Val Leu Thr Val Leu His Gln Asp Trp Leu Asn Gly Lys Glu Tyr
                325                 330                 335

Lys Cys Lys Val Ser Asn Lys Ala Leu Pro Ala Pro Ile Glu Lys Thr
            340                 345                 350

Ile Ser Lys Ala Lys Gly Gln Pro Arg Glu Pro Gln Val Tyr Thr Leu
        355                 360                 365

Pro Pro Ser Arg Asp Glu Leu Thr Lys Asn Gln Val Ser Leu Thr Cys
    370                 375                 380

Leu Val Lys Gly Phe Tyr Pro Ser Asp Ile Ala Val Glu Trp Glu Ser
385                 390                 395                 400

Asn Gly Gln Pro Glu Asn Asn Tyr Lys Thr Thr Pro Pro Val Leu Asp
                405                 410                 415

Ser Asp Gly Ser Phe Phe Leu Tyr Ser Lys Leu Thr Val Asp Lys Ser
            420                 425                 430

Arg Trp Gln Gln Gly Asn Val Phe Ser Cys Ser Val Met His Glu Ala
        435                 440                 445

Leu His Asn His Tyr Thr Gln Lys Ser Leu Ser Leu Ser Pro Gly Lys
    450                 455                 460
```